(19) Europäisches Patentamt
European Patent Office
Office européen des brevets

(11) **EP 2 442 894 B1**

(12) **EUROPEAN PATENT SPECIFICATION**

(45) Date of publication and mention
of the grant of the patent:
**11.04.2018 Bulletin 2018/15**

(51) Int Cl.:
*B01D 61/00* (2006.01)  *B01D 61/02* (2006.01)
*B01D 11/04* (2006.01)  *C02F 1/44* (2006.01)

(21) Application number: **10730198.8**

(22) Date of filing: **18.06.2010**

(86) International application number:
**PCT/GB2010/001191**

(87) International publication number:
**WO 2010/146365 (23.12.2010 Gazette 2010/51)**

(54) **BIOMIMETRIC MEMBRANES AND USES THEREOF**

BIOMIMETRISCHE MEMBRANEN UND IHRE VERWENDUNG

MEMBRANES BIOMIMÉTRIQUES ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **19.06.2009 DK 200900758**
**18.03.2010 US 315226 P**

(43) Date of publication of application:
**25.04.2012 Bulletin 2012/17**

(60) Divisional application:
**15178838.7 / 2 977 097**

(73) Proprietor: **Aquaporin A/S**
**2800 Kongens Lyngby (DK)**

(72) Inventors:
• **JENSEN, Peter, Holme**
**DK-2100 København (DK)**
• **HANSEN, Jesper, Sondergaard**
**DK-2860 Søborg (DK)**
• **VISSING, Thomas**
**DK-1750 København (DK)**
• **PERRY, Mark, Edward**
**DK-2100 København (DK)**
• **NIELSEN, Claus, Helix**
**DK-2630 Taastrup (DK)**

(74) Representative: **Nordic Patent Service A/S**
**Bredgade 30**
**1260 Copenhagen K (DK)**

(56) References cited:
EP-A1- 1 801 572       WO-A1-2009/076174
US-A- 3 637 488        US-A- 5 229 004
US-A1- 2007 105 094    US-A1- 2009 120 874
US-A1- 2009 120 874

**Description**

**FIELD OF THE INVENTION**

The present invention relates to the use of a liquid membrane systems suitable for water extraction, and more particularly to liquid membrane systems having biochannels, such as protein channels, incorporated into an amphiphilic vesicle bilayer for the extraction of water and/or small solutes from aqueous media. More particularly, the liquid membrane system is an aquaporin liquid membrane consisting of aquaporins in a vesicular dispersion of amphiphilic molecules, particularly for pure water extraction from aqueous liquid media, e.g. in forward osmosis applications. In addition, the present invention relates to a fluorescence assay for measuring the hydrophilicity of the membrane protein environment, wherein said assay is based on a fluorescently labelled membrane protein using an environmental sensitive fluorescent probe.

**BACKGROUND OF THE INVENTION**

Liquid membrane separation processes have been used for removal of dissolved substances such as ions from aqueous solutions, such as disclosed in US 4360448(A). This relates to a process for the removal of dissolved species from aqueous solutions, which comprises contacting said aqueous solution with an emulsion, said emulsion comprising an exterior phase which is characterized as being immiscible with said aqueous solution and yet permeable to said dissolved species, and an interior phase which contains a reactant, such as an ion exchange compound, capable of converting said dissolved species to a non-permeable form. The dissolved species permeate the exterior phase, into the interior phase where they are converted into non-permeable forms and thus retained in the interior phase of said emulsion. The aqueous solution, depleted in said dissolved species, is separated from said emulsion and the emulsion cycled for reuse. However, when multiple or unspecified ions or solutes are present in an aqueous solution or medium, such as a biological liquid it becomes increasingly complex to remove solutes by this or similar methods, since it would be necessary to device a specific reactant for each species to be removed. Another example of the use of a liquid membrane extraction process is described in (WO 87/002380) Production of Low-Ethanol Beverages by Membrane Extraction which relates to membrane extraction systems designed to selectively remove ethanol from wine and other beverages while retaining the water and numerous other organic constituents. Thus, liquid membrane separation methods have hitherto been developed for selective removal of solutes in, e.g. aqueous liquids. Seeing a need to selectively remove or extract water from aqueous liquid sources the present inventors have devised a liquid membrane process suitable to removal or extraction of pure water from an aqueous liquid using the selective water channel known from aquaporin proteins.

Fluorescent-based activity assays are well-established for soluble proteins, but not for membrane proteins. A likely reason for this is that membrane proteins are fragile when they are taken out of their natural environment - the biological membrane. Moreover, the accessibility to commercially available protein species has been restricted to only few membrane proteins. This is related to the difficulty in expressing and purifying membrane proteins in large quantities (gram-scale). Membrane proteins typically retain their function upon reconstitution into a biomimetic membrane that sufficiently mimics the protein's natural environment. There is today an unmet need for an assay for screening lipid membrane components for their usefulness in the creation of a biomimetic membrane formulation that meets the membrane protein requirements, i.e. specific hydrophilic and hydrophobic regions or layers. At the same time, such an assay would provide useful information about the folding state of a membrane protein. US 2009/0120874 discloses supported lipid bilayer membranes with aquaporin channels. In the oil phase however no squalene/squalane is used. EP1801572 discloses the use of squalene in the process of the formation of another lipid double layer membrane.

**SUMMARY OF THE INVENTION**

Broadly, the present invention relates to a liquid membrane system in the form of a biochannel containing bulk liquid membrane (BLM), biochannel containing emulsion liquid membrane (ELM), and biochannel containing supported (immobilised) liquid membrane (SLM), or a combination thereof, wherein the liquid membrane system is based on vesicles formed from amphiphilic compounds such as lipids forming a bilayer wherein biochannels have been incorporated and wherein said vesicles further comprise a stabilising oil phase. The liquid membrane system is an aquaporin containing liquid membrane system in accordance with claim 1, such as an aquaporin containing bulk liquid membrane (BLM), an aquaporin containing emulsion liquid membrane (ELM), and aquaporin containing supported (immobilised) liquid membrane (SLM), and/or combinations thereof, wherein said liquid membrane system comprises biochannels, such as aquaporin water channels, in a dispersion of amphiphilic molecules. The liquid membrane system may be used in a range of applications, including water extraction from liquid aqueous media by forward osmosis, e.g. for desalination of salt water.

Accordingly, in a first aspect, the present invention provides a liquid membrane system in the form of a biochannel containing bulk liquid membrane (BLM), biochannel containing emulsion liquid membrane (ELM), and biochannel containing supported (immobilised) liquid membrane (SLM), or a combination thereof, wherein the liquid membrane system comprises vesicles formed from one or more amphiphilic compounds forming a bilayer into which the biochannels have been incorporated and wherein the vesicles further comprise a stabilising oil phase.

In a further aspect, the present invention provides a method of extracting water from an aqueous liquid comprising the following steps:

a) mixing an amount of the liquid membrane system of any one of the preceding claims into a first aqueous liquid having an osmotic pressure which is less than that of the vesicles to form a suspension,
b) allowing the vesicles in said suspension to absorb pure water from said first liquid and expand as long as an osmotic pressure gradient exists,
c) separating the expanded vesicles the from the first liquid, and
d) resuspending said vesicles from step c) in a second aqueous liquid having an osmotic pressure that exceeds the pressure of the expanded vesicles to allow for the extracted water in the vesicles to flow into and dilute said second liquid as long as an osmotic gradient is present leaving the vesicles in a non-expanded state.

In a further aspect, the present invention provides an apparatus for pure water extraction from an aqueous liquid media which comprises one or more liquid membranes as described herein.

In a further aspect, the present invention provides solventless giant protein vesicle consisting essentially of an amphiphilic lipid, transmembrane protein channels, and an oil in an aqueous dispersion, wherein the lipid to protein molar ratio is in the range of from about 1:50 to about 1:400.

In a further aspect, the present invention provides compositions comprising the giant protein vesicles as described herein.

In a further aspect, the present specification describes methods of preparing the giant protein vesicles consisting essentially of an amphiphilic lipid, transmembrane protein channels, and an oil in an aqueous dispersion, and wherein the lipid to protein molar ratio is in the range of from about 1:50 to about 1:400, the method comprising the steps of:

a) preparing liposomes from a dried lipid solution that has been rehydrated in a detergent containing buffer and extruded through a filter of about 100 nm to about 500 nm pore size,
b) mixing the liposomes from a) with a transmembrane protein solution, wherein the protein is optionally linked to a fluorescent label,
c) dialysing the mixture from b) overnight using a molecular weight cut off of about 10kDa,
d) separating the proteoliposome vesicles formed in step c), e.g. by centrifugation,
e) optionally obtaining an absorbance spectrum of the GPVs formed which is compatible with the fluorescent label used in order to verify correct insertion of the transmembrane protein in the vesicle membranes, and
f) mixing the proteoliposomes obtained in step d) with a lipid in an oil phase solution containing the same lipid as in step a), e.g. in a molar ratio ranging from about 1:3 v/v to about 1:12 v/v,

thereby resulting in the formation of GPVs from the proteoliposomes.

In any particular case, within the general framework of the methods disclosed herein, the skilled person will be able to select appropriate specific conditions according to the size of the sample of giant protein vesicles being prepared and the properties of the reagents used.

By way of example, the conditions used in step c) employs a dialysate flow of from about 1 to about 10 mL/min, more preferably from about 1 to about 5 mL/min and most preferably about 3 mL/min.

By way of example, the separation in step d) uses centrifugation, for example between about 10,000-20,000 rpm (e.g. 14,200 rpm) for between about 1-5 minutes (e.g. about 90 sec).

By way of example, the mixing in step f) use end-over-end rotation overnight at about 4°C.

In a further aspect, the present application describes methods of preparing giant protein vesicles consisting essentially of an amphiphilic lipid, transmembrane protein channels, and an oil in an aqueous dispersion, and wherein the lipid to protein molar ratio is in the range of from about 1:50 to about 1:400, the method comprising self assembling the vesicles from an aqueous mixture of said amphiphilic lipid, transmembrane protein channels, and oil following end-over-end mixing.

In a further aspect, the present invention provides the use of the giant protein vesicles prepared according to a method as described herein for extraction of water through forward osmosis.

In a further aspect, the present invention provides the use of the giant protein vesicles prepared according to a method as described herein for re-extraction of pure water from a patient's plasma lost through haemodialysis.

In a further aspect, the present invention provides supported liquid membranes having an open or closed sandwich construction, wherein a substantially flat porous filter material provides support on one or both sides of a layer of proteoliposomes, thereby immobilizing the layer.

In a further aspect, the present invention provides a composite filter membrane or disk created by sandwiching a layer of aquaporin containing proteoliposomes or giant protein vesicles in between filter materials selected from ultrafiltration membranes, nanofiltration membranes and microfiltration membranes.

In a further aspect, the present invention provides a liquid membrane system in the form of a microemulsion comprising lipid vesicles having membrane bound or incorporated biochannels or protein channels, such as aquaporin water channels in an oil phase, and which is suitable for water extraction from liquid aqueous media.

In a further aspect, the present invention provides a method of preparing said microemulsion. Said liquid membrane system may further be contained or immobilised in a contactor module or in an essentially planar porous sandwich construction.

In a further aspect, the present invention provides an assay for measuring the hydrophilicity of the membrane protein environment, where said assay is based on a fluorescently labelled membrane protein using an environmental sensitive probe the fluorescence of which can be measured when the protein to which it is attached is reconstituted in a biomimetic membrane, such as a lipid bilayer membrane or corresponding amphiphilic membrane.

In a further aspect, the present specification describes a method for determining whether a specific combination of membrane-forming lipid and a membrane protein is capable of forming a biomimetic membrane in which the membrane protein is correctly folded, the method comprising:

(a) introducing a membrane protein labelled with a fluorescent label into a membrane forming lipid mixture, wherein a fluorescent property of the fluorescent label is dependent on the environment of the membrane protein in the biomimetic membrane formed by the combination of protein and lipid;
(b) detecting a fluorescent signal from the membrane protein labelled with a fluorescent label; and
(c) determining from the fluorescent signal whether the membrane the combination of lipid and membrane protein is capable of forming a biomimetic membrane in which the protein is correctly folded

In a further aspect, the present invention provides a method for determining whether a membrane protein is capable of folding correctly in a biomimetic membrane, the method comprising:

(a) introducing a membrane protein labelled with a fluorescent label into a system for determining whether the membrane protein is capable of folding correctly in a biomimetic membrane, wherein a fluorescent property of the fluorescent label is dependent on the environment of the membrane protein in the biomimetic membrane;
(b) detecting a fluorescent signal from a membrane protein labelled with a fluorescent label; and
(c) determining from the fluorescent signal whether the membrane protein is correctly folded in the biomimetic membrane.

In a further aspect, the present invention provides a biomimetic membrane for measuring the activity and/or function of membrane protein forming the biomimetic membrane, wherein the membrane protein comprises a fluorescent label having a fluorescent property of the fluorescent label is dependent on the environment of the membrane protein in the biomimetic membrane, wherein the activity or function of the membrane protein is measurable by detecting a fluorescent signal from the fluorescent label.

In a further aspect, the present invention provides a sensor comprising a biomimetic membrane as described herein.

Embodiments of the present invention will now be described by way of example and not limitation with reference to the accompanying figures and examples.

**BRIEF DESCRIPTION OF THE DRAWINGS**

Fig. 1 shows the general principles of three types of liquid membranes: Bulk liquid membrane (BLM), Emulsion liquid membrane (ELM), Supported liquid membrane (SLM).
Fig. 2 shows hollow fibers and spiral wound separation modules in liquid membrane modules.
Fig. 3 shows a principle sketch of a two Module Hollow Fiber Supported Liquid Membrane.
Fig. 4 shows a principle sketch of an apparatus and a method for concentration of aqueous solutions using the vesicles of the invention.
Fig. 5 shows a principle sketch of a method of providing a nutrient drink comprising pure drinking water through forward osmosis.

Fig. 6 shows a principle sketch of the use of the vesicles of the invention in forward osmosis - following ultrafiltration of a uniform electrolyte. This is an example of a complete application of water extraction from any aqueous solution or liquid.

Fig. 7 shows a principle sketch for the use of vesicles of the invention in pressure retarded osmosis for osmotic power production.

Fig. 8 shows the relation between liquid membrane formulation and concentration of the proteoliposome microemulsion (liquid membrane).

Fig. 9 is a drawing illustrating the basic parameters for calculating .

Fig. 10 is a schematic drawing of a forward osmosis batch cell unit.

Fig. 11 is a drawing of a filter cup for forward osmosis.

Fig. 12 is a graph showing difference of osmolarity over time between draw and feed solutions.

Fig. 13 is a microscope image of a liquid membrane preparation of DOPC vesicles having SoPIP2;1 proteins incorporated in its membrane with a scale bar of 200 $\mu$m shown for comparison.

Fig. 14 is a fluorescence image is of aquaporin SoPIP2;1 labeled with the fluorophore badan™ reconstituted into giant protein vesicles.

Fig. 15 is a graph showing forward osmosis flow QA of a SoPIP2;1 GPV formulation.

Fig. 16 With same configurations listed for Fig. 15, a non-aquaporin /empty formulation is shown.

Fig. 17 shows the secondary structure of AqpZ tetramer with four badan™ labels attached.

Fig. 18 shows fluorescence spectroscopy spectra of badan-SoPIP2;1 and badan-AqpZ.

Fig. 19 shows the normalized intensity of fluorescence spectra as a function of wavelength.

Fig. 20 illustrates GP ratios for two different aquaporins reconstituted in two different lipid vesicles.

Fig. 21 shows a schematic showing the salt gradient and counter-current of a normal kidney mimicked by the liquid membrane of the present invention.

## DETAILED DESCRIPTION OF THE INVENTION

The aquaporin containing liquid membrane system described herein may be in the form of an aquaporin containing emulsion liquid membrane (ELM), wherein said liquid membrane comprises aquaporin water channels in a dispersion of amphiphilic molecules, preferably comprising vesicles in the form of proteoliposomes, having functional aquaporins incorporated into an amphiphilic vesicle bilayer, such as a lipid bilayer and the like.

The aquaporin containing emulsion liquid membrane, or Aqp-ELM, of the present invention can be used for water extraction by being mixed into or suspended in a first aqueous liquid having an osmotic pressure which is less than that of the Aqp-ELM vesicles which will selectively transport pure water molecules from said first liquid through the aquaporin water channels into the swelling vesicles. After extraction of pure water from the first liquid the vesicles can be separated, e.g. by centrifugation or filtration, and resuspended in a second aqueous liquid having an osmotic pressure that exceeds the pressure of the vesicles having extracted pure water. The extracted water will now flow from the shrinking vesicles into the second liquid as long as an osmotic gradient is present. Second aqueous liquids should preferably be separable from the product (purified) water, have low or no toxicity, and be chemically inert to the liquid membranes. Examples of second aqueous liquids (draw solutions) are mixtures of glucose and fructose that have been used for seawater desalination, and lately draw solutions based on combining ammonia and carbon dioxide gases in specific ratios in highly concentrated draw solutions of thermally removable ammonium salts have been obtained, cf. J.O. Kessler, and C.D. Moody, Drinking water from sea water by forward osmosis, Desalination 18 (1976) 297-306; J.R. McCutcheon, R.L. McGinnis, and M. Elimelech, Desalination by a novel ammonia-carbon dioxide forward osmosis process: influence of draw and feed solution concentrations on process performance. J. Membr. Sci. 278 278 (2006) 114-123), and Method and apparatus for producing potable water from seawater using forward osmosis By Kirts, Richard Eugene. Alternatively, water can be easily separated from the diluted draw solution by heating near 60°C to yield fresh water, ammonia and carbon dioxide. Both the ammonia and carbon dioxide can then be reused as solutes for the draw fluid, cf. Low (2009).

U.S. Pat. Appl. Publ. (2009), US 2009308727 A1 20091217 discloses a method and apparatus for desalinating seawater which uses an ammonia-bicarbonate forward osmosis desalination process. Seawater is pumped through one side of a membrane assembly, and a draw solution is pumped through the other side of the membrane assembly. The draw solution withdraws water molecules from the seawater through the membrane into the draw solution, and a draw solution separator receives a heated draw solution which then decomposes into ammonia, $CO_2$ and water. Potable water is separated from ammonia and $CO_2$ gas. Subsequently, the ammonia gas and $CO_2$ gas are recombined with a portion of the potable water stream to reform the ammonium bicarbonate draw solution. One embodiment of the present invention relates to the use of the liquid membrane system of the invention in a method and an apparatus as disclosed in US 2009308727 A1. In another embodiment of the present invention, the aquaporin containing liquid membrane system is used in water reextraction from the dialysate resulting from haemodialysis. There are at least two useful applications of the liquid membrane system of the invention in improvement of haemodialysis methods:

> i) Production of ultrapure water as described herein can replace the very elaborate systems for water purification that are presently necessary in order to restore the water content of the patients plasma.
> ii) Following the forward osmosis process used when creating the dialysate large amounts of water stemming from the patient's blood plasma is simultaneously removed,and this may be extracted using an aquaporin liquid membrane in any of the methods described herein.

The aquaporin containing vesicles (Aqp-ELM) described herein are able to swell and shrink in repeated cycles. Typically the vesicles are pre-shrunk before they are brought into contact with a first aqueous liquid having a lower osmotic pressure than the vesicles' interior, and from which it is desirable to extract water. Following separation of the swelled vesicles from said first aqueous liquid it is possible to extract the ab sorbed water into a draw solution having a higher osmotic pressure than the interior of the swelled vesicles. It has been shown that the volume of DOPC vesicles containing gramicidin A channels may swell up to about 16 % by water transport, cf. M. Goulian et al., Biophysical Journal, Vol. 74, January 1998, pp. 328-337. It has also been shown for gel-filled DOPC vesicles that the volume may shrink with up to about 80% of the initial volume, cf. A. Viallat et al. Biophysical Journal, Vol. 86, April 2004, pp. 2179-2187. In one aspect of the invention the aquaporin containing vesicles are in the form of solventless prepared giant protein vesicles (GPV). The GPVs and the method of preparing them are disclosed herein for the first time. The GPVs of the invention are characterized in being prepared using oil stabilization instead of a solvent. In addition the GPVs of the invention may be prepared without the use of electroformation. Moreover the protein content can be precisely determined prior to the actual vesicle formation by mol/mol, fraction, i.e. by fluorescence spectroscopy, where the signal intensity is directly correlated with protein content.

Small unilamellar phosphatidylcholine (SUV) vesicles having a diameter of approx. 20 nm are osmotically sensitive. Such vesicles respond to osmotic pressure by swelling or shrinking depending on the direction of the applied salt gradient. This is true for small unilamellar vesicles of egg phosphatidylcholine and dimyristoylphosphatidylcholine below and above their crystal-to-liquid crystal transition temperature. In the presence of osmotic gradients, the influx and efflux of $H_2O$ is de-coupled with the movement of ions due to the presence of aquaporins. During osmotically induced shrinking and swelling of SUV the integrity of the phospholipid bilayer is maintained to the extent that vesicles do not break, and therefore equilibration between external medium and vesicle cavity does not take place unless the membranes contain aquaporin proteins.

Typical osmotic pressures of the first aqueous liquid or source phase is in the range of about 100 mOsm to about 500 mOsm or 1000 mOsm, and typical osmotic pressures of the second aqueous liquid or receiving phase are about 100 to 1000 mOsm higher in order to obtain a suitable osmotic pressure difference. The osmolality of sea water ranges from 2000-2400 mOsm, primarily contributed by sodium chloride. This is 8 times the normal osmolality of blood plasma, which is about 275-299 milli-osmoles per kilogram. The most concentrated urine our kidneys can produce ranks at 1400 mOsm, far below the level of ocean water.

In addition, the invention relates to a liquid membrane system, such as in the form of an emulsion liquid membrane (ELM) wherein said liquid membrane comprises lipid vesicles having membrane incorporated amphiphilic molecules in a dispersion with biochannels, preferably in the form of a vesicle dispersion, e.g. in the form of proteoliposomes, having biochannels, e.g. aquaporins, incorporated into an amphiphilic vesicle bilayer, such as a lipid bilayer, an amphiphilic block-copolymer, i.a. of the formulae AB-BA, ABA, or ABC, or a hybrid amphiphilic membrane such as in liposomes based on PEG conjugated amphiphiles (e.g. polyethylene glycol-phosphatidylethanolamine conjugates (PEG-PE)), and the like. Besides aquaporins said biochannels comprise boron nitride nanotubes, carbon nanotubes, and amphiphilic pore forming molecules and transmembrane channel molecules selected from the group consisting of transmembrane proteins, i.a. beta-barrel pores such as alpha-hemolysin and OmpG, FomA, VDAC; transmembrane peptide pores (alamethicin, valinomycin, gramicidin A) including synthetic peptides, ion channels, as reviewed by Boon, M. and Smith, BD; 2002 ("Synthetic membrane transporters". Current Opinion in Chemical Biology 2002, 6:749-756), and ion-selective ionophores such as sodium selective ETH 157, potassium selective SQI-Pr and valinomycin, chloride ionophore Trioctyltin chloride and the like.

The proteoliposomes of the invention are stabilised by the creation of lipid vesicles (or GPV particles) in an enriched oil phase. One way of doing this is to exchange the extra-vesicular water by other solvents. To provide a solvent environment which does not perturb the GPV bilayer or which has a reduced tendency of perturbing the GPV bilayer, an oil phase comprising non-polar solvents can be exchanged for compounds that exhibit a higher degree of hydrophilicity, or non-polar solvents of large molecular sizes can be chosen. Compounds of low toxicity are also preferred. Natural oil compounds are known to form relatively stable lipid emulsions exhibiting physical stability and being non-toxic. According to the invention these oils are squalene, squalane, or a mixture thereof. Squalene is an example of a hydrocarbon solvent whereby black lipd membranes' can be made 'solvent-less' [White S., Biophys. J., vol. 23, 1978]. It is preferred in this invention to reduce the content of organic solvents that are normally employed in lipid vesicle emulsions to obtain a solvent less or even solvent free vesicle com position.

In a further aspect, the present specification also describes to a fluorescence assay, e.g. using a naphthalene derivative fluorescent molecule, e.g. the fluorophore 6-bromoacetyl-2-dimethylaminonaphthalene (badan™) as a probe which can be covalently attached to Cys residues of a protein. The probe is sensitive to the hydrophilicity of the molecular environment to which it is exposed giving a maximum fluorescence emission range of 450 nm to 550 nm. When the badan probe is hydrophobic exposed it has its highest fluorescence emission yield and this is at 450 nm. In contrast, when the badan probe is hydrophilic exposed (e.g. water) it has low fluorescence emission yield and emission maximum at around 550 nm. Intermediate hydrophilic/hydrophobic conditions to those explained above give rise to emission maximum and fluorescence yield in between the above described features of badan fluorescence properties. Table 1 below is a list of useful fluorescent probes.

**Table 1**

| Abbreviation: | Chemical name: |
| --- | --- |
| Badan | 6-Bromoacetyl-2-dimethylaminonaphthalene |
| Acrylodan | 6-acrylolyl-2-d imethylam ino-naphthalene |
| Laurdan | 6-Dodecanoyl-2-dimethylaminonaphthalene |
| Prodan | 6 - Propionyl-2-dimethylaminonaphthalene |
| 1,5-IAEDANS | 5-((((2-iodoacetyl)amino)ethyl)amino)naphthalene-1-sulfonic acid |
| IAANS | 2-(4'-(iodoacetamido)anilino)naphthalene-6-sulfonic acid |
| MIANS | 2-(4'-maleimidylanilino)naphthalene-6-sulfonic acid, sodium salt |

This assay provides information useful for measuring and/or imaging of the partitioning of a membrane protein in amphiphilic biomimetic membranes and, thus, the folding state of a membrane protein. The type of information obtained through the assay depends on the type of membrane association specific to the membrane protein in question and the position of the fluorescent probe on the protein. An exemplary embodiment of the invention is the fluorescence labelling using badan™ on an aquaporin molecule, where the degree of successful protein reconstitution into biomimetic membrane is measured by either spectroscopy or imaged by microscopy.

This assay is specifically useful in optimisation of the reconstitution processes of the protein, and/or in the selection of the biomimetic membrane formulation (phospholipids and other bulk membrane constituents such as cholesterol and the like) in order to fit the specific requirements of individual membrane proteins, such as aquaporins.

In addition the method is useful in creating membrane protein sensors based on fluorescence labelling and to create an assay of measuring hydrophilic/hydrophobic exposure of membrane proteins. The present invention also provides a novel methodology of creating fluorescence based membrane protein sensors for measuring membrane protein activity.

**Definitions**

The term liquid-liquid extraction is used for a separation process using liquid membranes. In the present invention this is a liquid-water extraction, as water is extracted into a liquid membrane.

Using a liquid membrane the general term "water extraction" will be used herein together with the general term "water separation".

The terms "vesicle" and the synonym "liposome" is used herein to specify approximately globular liquid structures of amphiphilic compounds, such as phospholipids. When proteins are incorporated in the vesicles the term is used interchangably with the term "proteoliposome" which has incorporated amphiphilic proteins, e.g. transmembrane proteins including aquaporins. A liposome can be formed at a variety of sizes as a uni-lamellar or multi-lamellar construction, where a lamella is one amphiphilic bilayer. Thus, there are several types of vesicles or liposomes which are all covered by the invention: MLV (multilamillar vesicles), SUV (Small Unilamellar Vesicles) and LUV (Large Unilamellar Vesicles) or GUV (Giant Unilamellar Vesicles). Amphiphilic molecules such as phospholipids can assemble themselves into tiny spheres, smaller than a normal cell, either as bilayers or monolayers. The bilayer structures are vesicles or liposomes. The monolayer structures are called micelles. In the context of this invention the term "proteoliposome" is not intended to refer to liposomes consisting primarily of globular lipid structures used for encapsulation of proteins, typically for drug delivery purposes. The vesicles of the invention are characterised in having an amphiphilic membrane which is suitable for insertion of and integration of a transmembrane protein. Preferably, the transmembrane proteins retain their native three-dimensional conformation when integrated in the vesicle membrane, and thus, the protein retain its functionality.

An especially common form of the vesicles of the invention is a "giant protein vesicle" or GPVs of relatively uniform size having a diameter in the range of from between about 20 to 25 μm to about 400 to about 500 μm and up to about 1000 μm and a typical average diameter in the range of between about 100 to about 250 μm.

The vesicles for the forming of the GPV's of the invention are preferably unilammellar vesicles of uniform size of a suitable lipid mixture, conveniently made by extrusion through track-etched polycarbonate filters, cf. "Vesicles of variable sizes produced by a rapid extrusion procedure", L.D. Mayer, M.J. Hope and P.R. Cullis, Biochimica et Biophysica Acta 858 (1986) 161-168, "Effect of Extrusion Pressure and Lipid Properties on the Size and Polydispersity of Lipid Vesicles", D.G. Hunter and B.J. Frisken, Biophysical Journal 74(6) 1986, 2996-3002, "Osmotic properties of large uni-lamellar vesicles prepared by extrusion." B L Mui, P R Cullis, E A Evans, and T D Madden, Biophysical Journal 64(2) 1993, 443-453. Preferred pore-size of extrusion filters are 0.200 microns yielding vesicles of about 0.15 +-0.06 μm. This does not exclude the use of smaller or larger pore size filters. Other methods exists for vesicle or liposome preparation, e.g.,. by an ether vaporization method, cf. D. Deamer and A. D. Bangham, Biochimica et Biophysica Acta (BBA) - Biomembranes Volume 443, Issue 3, 7 September 1976, Pages 629-634, and several methods have been described for the preparation of small unilamellar vesicles of approximately 250 Angstrom in diameter (Szoka, F. & Papahadjopoulos, D. (1980) Annu. Rev. Biophys.Bioeng. 9, 467-508) produced by ultrasonic irradiation; controlled removal of detergent, e.g., bile salts or Triton X-100, from aqueous dispersions of detergent/phospholipid micelles by gel filtration or dialysis; injection of phospholipid solution in ethanol, or other solvent, into water and removal of the organic solvent by evaporation; and extrusion of unsonicated aqueous phospholipid dispersions at high pressure through a French press. However, it is shown herein for the first time that giant protein vesicles may be prepared without the use of electroformation.

The term "lipid" as used herein covers preferably amphiphilic lipids, e.g. phospholipids, phosphoglycerides, sphingolipids, and cardiolipin, as well as mixtures thereof, e.g. phospholipids such as 1,2-dipalmitoyl-sn-phosphatidylcholine (DPPC), or mixtures of phospholipids. Useful lipids are listed in Table 1 in WO 2006/122566 which is incorporated herein by reference.

The term "biochannel" as used herein shall mean any membrane spanning channel or pore, such as protein channels that can be incorporated into an amphiphilic vesicle bilayer for the extraction of water and/or small solutes from a liquid aqueous medium. Also included in the term are pore forming membrane inclusions, such as functional protein channels, e.g. aquaporins, incorporated into an amphiphilic vesicle bilayer, such as a lipid bilayer, a bilayer formed by self assembly from an amphiphilic block-copolymer, i.a. of the formulae AB-BA, ABA, or ABC, or a hybrid amphiphilic membrane such as in liposomes based on PEG conjugated amphiphiles (e.g. polyethylene glycol-phosphatidylethanolamine conjugates (PEG-PE)), and the like. The term may also comprise nanotubes that are shown to support water transport, e.g. in a single file of water molecules, i.e. certain boron nitride nanotubes and carbon nanotubes. Further included in the term are other amphiphilic pore forming molecules including transmembrane channel molecules selected from the groups consisting of transmembrane proteins, i.a. beta-barrel pores such as alpha-hemolysin and OmpG, FomA, VDAC, transmembrane peptide pores (alamethicin, valinomycin, gramicidin A), ion channels such as potassium or sodium channels, and the like.

The term "aquaporin" as used herein shall mean any functional water channel, such as the transmembrane proteins described in WO/2006/122566 "Membrane for filtering of water" and by Tamir Gonen and Thomas Walz, Quarterly Reviews of Biophysics (2006), 39:4:361-396, Cambridge University Press. A preferred aquaporin protein as used herein is selected from the group consisting of Aqp 4, Aqp 1, Aqp Z, SoPIP2;1 and monomeric, dimeric, tetrameric and higher oligomers as well as functional variants thereof including mutated, conjugated and truncated versions of the primary sequence.

The terms "aqueous liquid" and "aqueous liquid media" are used herein to encompass aqueous solutions; natural water sources; liquids of biological origin such as fruit and vegetable juices, blood, milk and urine; waste water sources; aqueous suspensions, dispersions, emulsions and the like.

The term "osmotic pressure" as used herein shall mean the pressure generated by the osmotic flow of water from an aqueous liquid through a semi-permeable membrane into a compartment containing aqueous solutes at a higher concentration. Potential osmotic pressure is the maximum osmotic pressure that could develop in a solution when separated from distilled water by a selectively permeable membrane. The potential osmotic pressure is determined by the number of solute "particles" in a unit volume of the solution as described by the van't Hoff equation.

The term "forward osmosis" (FO) signifies a process where the osmotic pressure differential across a semi-permeable membrane is the driving force for transport of water through the membrane. The FO process results in concentration of a feed stream and dilution of a highly concentrated stream (referred to as the draw solution), cf. Cath et al., Journal of Membrane Science, 281 (2006) 70-87.

The term "first aqueous liquid" corresponds to "feed" liquid or the source phase.

The term "second aqueous liquid" corresponds to "draw" liquid or the receiving phase, also known as stripping solution.

The term "standard form factors" usable with liquid membranes as described herein shall mean the modern industry device and apparatus standards for liquid membrane extraction equipment.

The term "liquid membrane contactor" as used herein shall mean a device or composition that will allow two liquid phase to come into contact with each other for the purpose of mass transfer between the phases, through an aquaporin bulk liquid membrane. Examples of contactors as used herein include two module hollow fiber modules, multibundle hollow fiber contactors, such as Liqui-Cel™ contactors, a Hollow fiber pertractor and a Two chamber contactor system, cf. http://sschi.chtf.stuba.sk/MembraneLab/Equipment.htm

The term "correctly folded" relates herein to the secondary and tertiary structure of membrane associated proteins, such as transmembrane proteins and the like. A correctly folded protein folds in its native secondary structure according to whether it is an alpha-helix, beta-barrel or mixed membrane protein, and the subunits of multimeric proteins, such as the tetrameric aquaporins, fold together to form the tertiary structure providing that the required environmental conditions are fulfilled, e.g. polarity, hydration, hydrophilicity, hydrophobicity, or amphiphilic properties. Correct folding is necessary for the function and activity of these proteins.

**Specific embodiments**

Use of the aquaporin liquid membrane of the invention is especially advantageous in production of fresh water from desalination of saline feed solutions, such as sea water, where the specific pure water transporting and chloride rejecting properties of the aquaporin water channels offer unique process conditions. An interesting embodiment of the invention is the use of aquaporin liquid membranes (e.g. emulsion liquid membranes, supported emulsion liquid membranes, or bulk liquid membranes) in a forward osmosis process for the production of fresh water, where salt water is the feed and a $CO_2/NH_3$ containing aqueous solution is the draw solution having the advantage of easy elimination of the dissolved gases through heating to about 58 °C, cf. McGinnis and Elimelech, Desalination, 207 (2007) 370-382; and Quirin Schiermeier, "Purification with a pinch of salt", Nature, 452, 20 March 2008.

Examples of liquid membranes of the invention in the form of aquaporin proteoliposomes are illustrated in the tables below:

**Table 2. Membrane surface per liter ELM / BLM for two different vesicle diameters**

| 200 nm proteoliposome | 400 nm proteoliposome |
|---|---|
| Max. ~$500.000^3$ liposomes/liter | Max. ~$250.000^3$ liposomes/liter |
| $A = 4 \pi r^2$ | $A = 4 \pi r^2$ |
| $12{,}56 \times 10^{-14}$ m$^2$ membrane surface/liposome | $50{,}24 \times 10^{-14}$ m$^2$ membrane surface/liposome |
| Max. ~15.700 m$^2$ membrane surface/liter | Max. ~7.850 m$^2$ membrane surface/liter |
| Max. transport rate/bar: ~66 liter/second ($p_f$ ~$10^{-13}$) | Max. transport rate/bar: ~33 liter/second ($p_f$ ~$10^{-13}$) |

**Table 3. Components per liter ELM / BLM for two different vesicle diameters**

| 200 nm proteoliposome | 400 nm proteoliposome |
|---|---|
| ~34.5 g aquaporin/liter membrane (50% coverage) | ~17.25 g aquaporin/liter membrane (50% coverage) |
| ~35 g lipid/liter membrane | ~17.5 g lipid/liter membrane |
| ~0,523 liter water/liter membrane (inside) | ~0,523 liter water/liter membrane (inside) |
| ~0,4 liter other component (outside) | ~0,4 liter other component (outside) |

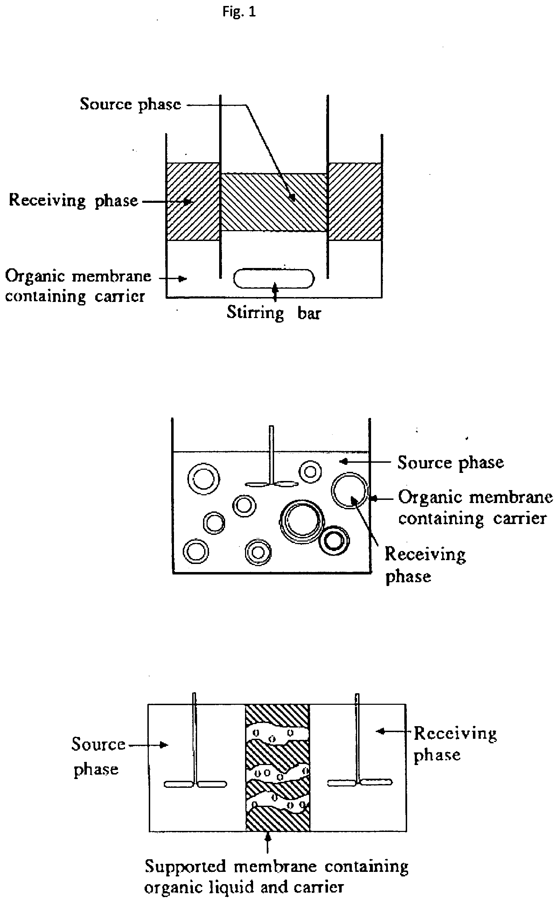

**The invention is illustrated in the figures 1 to 19 which are explained in detail below:**

Fig. 1 shows the general principles of three types of liquid membranes: Bulk liquid membrane (BLM), Emulsion liquid membrane (ELM), Supported liquid membrane (SLM). In the case of aquaporin liquid membranes, the carrier consists of aquaporin water channels embedded into an amphiphilic vesicle bilayer, such as a phospholipid bilayer and the like. For all three liquid membrane types, the source phase and the receiving phase will be an aqueous solution, where the receiving phase has a higher osmotic gradient than the source phase.

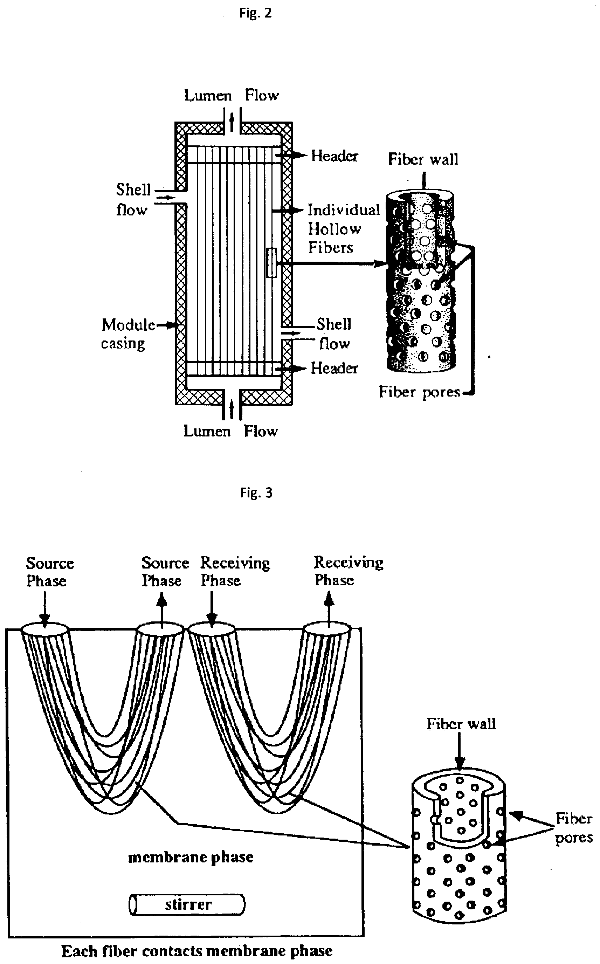

Fig. 2 shows hollow fibers and spiral wound separation modules in liquid membrane modules. For both modules the build up works as a contactor, between a source phase and a receiving phase. The source phase and the receiving phase will both be an aqueous solution, where the receiving phase has a higher osmotic gradient than the source phase.

Fig. 3 shows a principle sketch of a two Module Hollow Fiber Supported Liquid Membrane with a bulk liquid membrane as the carrier. In the case of an aquaporin bulk liquid membrane being the carrier, the aquaporin bulk liquid membrane will extract water from the source phase through the aquaporin water channels and into the receiving phase. The source phase and the receiving phase will both be an aqueous solution, where the receiving phase has a higher osmotic gradient than the source phase.

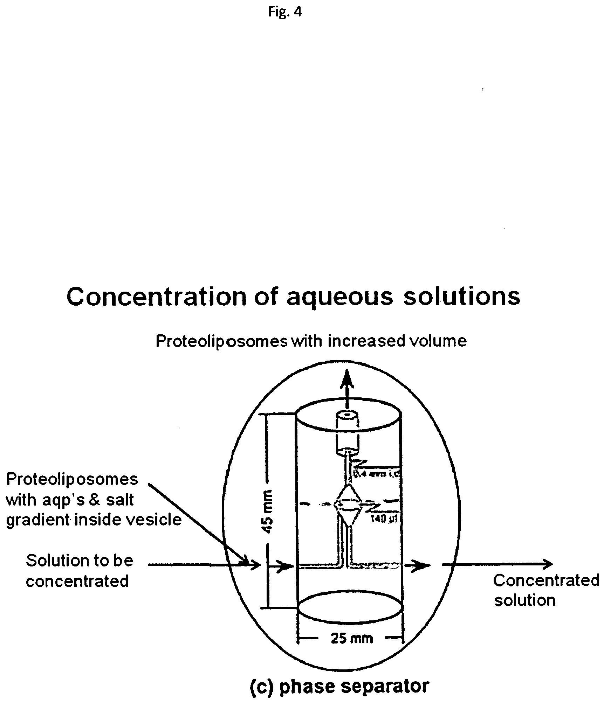

Fig. 4 shows a principle sketch of an apparatus and a method for concentration of aqueous solutions using the vesicles of the invention. In the case of an aquaporin emulsion liquid membrane being the carrier, the aquaporin emulsion liquid membrane will extract water from the solution to be concentrated, into the aquaporin emulsion liquid membrane, thereby ending up with a concentrated solution.

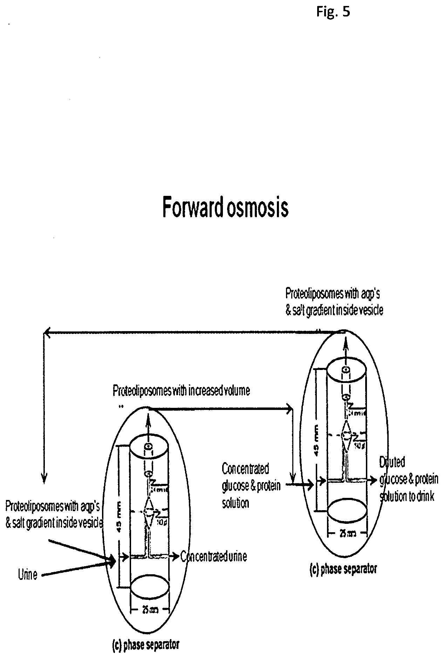

Fig. 5 shows a principle sketch of a method of providing a nutrient drink comprising pure drinking water through forward osmosis using an aquaporin emulsion liquid membrane as the carrier system. As an example, the aquaporin emulsion liquid membrane will extract water from a urine solution into the aquaporin emulsion liquid membrane. The aquaporin emulsion liquid membrane and the concentrated urine solution will be phase separated, and following the aquaporin emulsion liquid membrane will be mixed with a receiving aqueous solution with a higher osmotic gradient. Water will then be extracted from the aquaporin emulsion liquid membrane into the receiving solution, and the aquaporin emulsion liquid membrane and the receiving solution will be phase separated, giving an end result of transfer of water from a urine solution to another solution, in this example being a solution of glucose and protein.

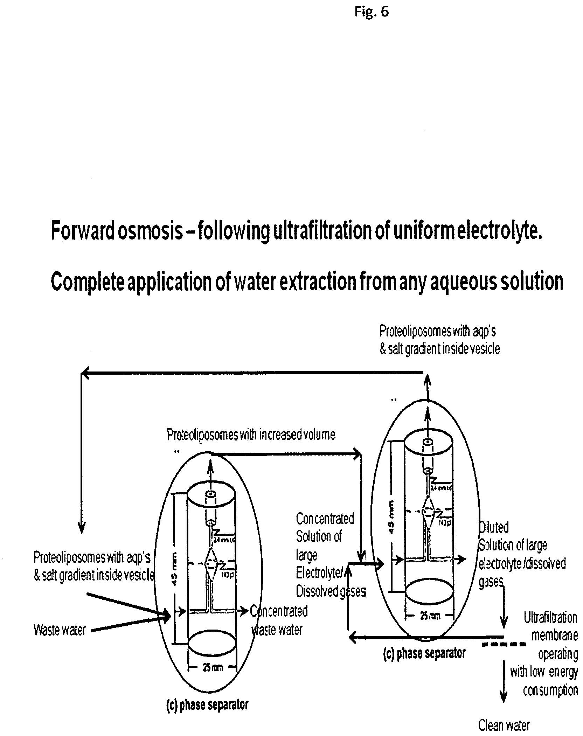

Fig. 6 shows a principle sketch of the use of the vesicles of the invention in forward osmosis, following ultrafiltration of a uniform electrolyte or degassing of dissolved gasses. This is an example of a complete application of water extraction from any aqueous solution or liquid. As an example, the aquaporin emulsion liquid membrane will extract water from a waste water solution into the aquaporin emulsion liquid membrane. The aquaporin emulsion liquid membrane and the concentrated waste water solution will be phase separated, and following the aquaporin emulsion liquid membrane will be mixed with a receiving aqueous solution with a higher osmotic gradient. Water will then be extracted from the aquaporin emulsion liquid membrane into the receiving solution, and the aquaporin emulsion liquid membrane and the receiving solution will be phase separated, giving an end result of transfer of water from a waste water solution to another solution, in this example being a solution of another electrolyte or a solution of dissolved gasses.

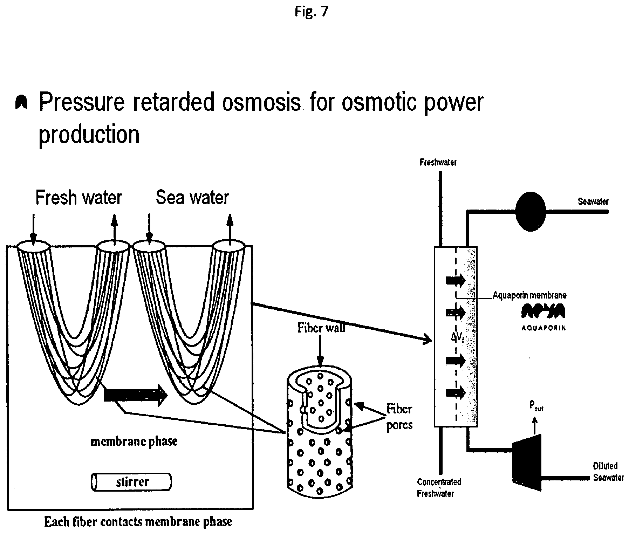

Fig. 7 shows a principle sketch for the use of vesicles of the invention in pressure retarded osmosis for osmotic power production. The example shows the principle sketch of a two Module Hollow Fiber Supported Liquid Membrane with a bulk liquid membrane as the carrier integrated into a pressure retarded osmosis system for osmotic power production. In the case of an aquaporin bulk liquid membrane being the carrier, the aquaporin bulk liquid membrane will extract water from the source phase through the aquaporin water channels in the aquaporin bulk liquid membrane and into the receiving phase. The source phase will in the example of pressure retarded osmosis be the brackish water/fresh water and the receiving phase will be seawater or even a brine of seawater. The extraction of water from brackish water into seawater will enable the production of osmotic power harvested through a turbine driven by a pressure gradient.

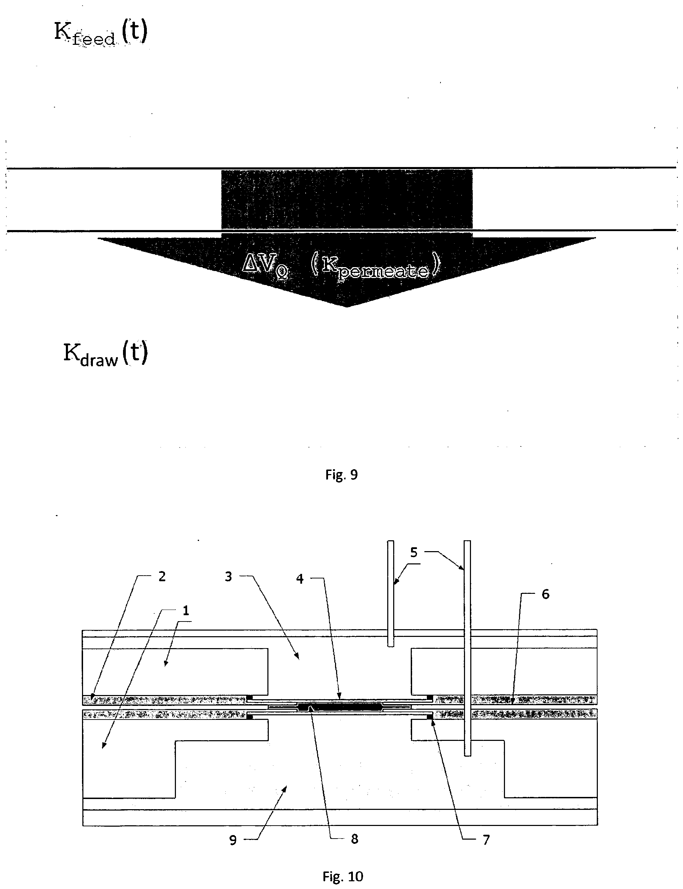

Fig. 9 In this schematic illustration of a forward osmosis unit cell the $K_{feed}$ (t) is used for measured conductivity of the feed solution, $K_{draw}$ (t) is used for measured conductivity of the draw solution, $\Delta V_Q(K_{permeate})$ is used for the volume of measured flow from feed. The broad arrow indicates direction of flow through the liquid membrane space.

The following formulae can be used in measurements of flux and calculation of rejection rate across the forward osmosis batch cell unit.

Flow: $Q(t) \approx \Delta V(t)$ where $\Delta V(t)$ is read from a measuring pipette

$$J(t) \approx \frac{Q(t)}{A}$$

Flux is calculated as: where A is liquid membrane contact area

$$J(t) \approx \frac{Q(t)}{A * P_{osmotic}}$$

Flux as function of osmotic pressure:

Osmotic pressure calculation for a solution of individually moving solute molecules:

$\Pi = c\,R\,T$ [chapt 2.11 in 'Quantities, Units and Symbols in Physical Chemistry,' INTERNATIONAL UNION OF PURE AND APPLIED CHEMISTRY, 1993]

c : molarity of individual moving solute

G: gas constant, $0.08206\ L \cdot atm \cdot mol^{-1} \cdot K^{-1}$

T: absolute temperature

For solutions deviating from ideality, the above equation becomes:

$$\Pi = \phi\, c\, R\, T$$

where $\phi$ is the osmotic coefficient. For a 0,15 M sucrose solution at 25 deg celsius $\phi = 1,01$ . [Sten-Knudsen, 'Stoftransport, membranpotentialer og elektriske impulser over biologiske membraner' Akademisk Forlag 1995]. $\phi$ may be determined from osmometry for particular solutions.

Example:

Osmotic pressure (in bar) of a 0,2 M sorbitol (D-sorbitol) at ambient temperature of 22 deg celsius:

$\Pi = \phi\, c\, R\, T \sim 1 \cdot 0{,}2\ \text{mole} \cdot L^{-1} \cdot 0.08206\ L \cdot \text{atm} \cdot \text{mol}^{-1} \cdot K^{-1} \cdot 295\ K \cdot 1{,}01325\ \text{bar} \cdot \text{atm}^{-1}$
$= 9{,}9\ \text{bar}$
$\phi$ is here assumed to be $\approx 1$.

Salt rejection, cf. Fig. 9:

By using draw and feed solutions containing fully ionized, strong electrolytes, the conductivity, $\kappa$, is here used as a simple concentration measure. An increase in draw solution conductivity reflects the amount of permeated ions in permeate volume, $Q(t)$, diluted into the draw volume ($V_0$ is initial draw volume):

$$\kappa_{permeate}(t) = \Delta\kappa_{draw}(t) \cdot \frac{V_0 + Q(t)}{Q(t)}$$

$$R = 1 - \frac{\kappa_{permeate}(t)}{\kappa_{feed}(t)}$$

A bulk R ('salt rejection') may be defined as:

Results:

In an experiment using an AQP liquid membrane prepared according to Example 1 in a forward osmosis batch unit cell where a 20 bar D-sorbitol draw solution was used the following salt rejection values were obtained (**Table 4**):

| t(min) | R |
|--------|------|
| 3 | 1,00 |
| 7 | 1,00 |
| 33 | 0,93 |

Fig. 10 shows an application of a use according to the invention where water extraction is achieved by forward osmosis from a feed solution to a draw solution through a composite filter membrane or disk created by sandwiching a proteoliposome layer in between selected filter materials, such as those listed in Table 3. In Fig. 10, the forward osmosis setup consists of two parts (upper and lower) that can be fitted around a central part containing the liquid membrane solution (8 - vesicles with aquaporin proteins) which is injected in between two filter membranes (4) via the injection channels (6). The volume in between the membranes is defined by the spacer/membrane holder (2). The clamping system (1) in connection with the O-rings (7) and screws ensure a tight fit and thus a sealed system. It also contains the feed solution compartment (3) and a draw solution compartment (9) that can both be equipped with means for stirring (not shown). The compartments can be accessed via level measuring tubes (5).

Assembly of housing unit:

1/ Place a magnetic stirrer bar inside the Draw solution compartment (9).
2/ Place two filters/membranes (4) on each side of the 'balcony' of the Membrane holder / Spacer part (2). Position the two O-rings on each side (7), outside each filter, sealing the liquid membrane compartment (8). Position this membrane-sandwich on the Draw solution compartment part and place the Feed compartment on top. Clamp and tighten the whole unit.

3/ Use the level measuring tubes to fill up the draw compartment with appropriate liquids. Inject liquid membrane sample into liquid membrane compartment. Fill up the feed compartment with appropriate liquid.

Example of the use of housing unit shown in Fig. 10 for a forward osmosis process across an aquaporin containing liquid membrane.

In an assembled unit two Alfa Laval nanofiltration membranes, Alfa Laval-NF (code 517819), is used as support of the liquid membrane compartment. For draw solution use a 0.8 M sorbitol (D-sorbitol, 85529 Sigma BioUltra). Sample volume is 300 $\mu$L of a liquid membrane emulsion, as described in Experimental section, Example 6. A phosphate buffered saline (PBS) solution, Sigma P-5368 (0.138 M NaCl, 0.0027 M KCl, 0.01 M of mono- and dibasic potassium phosphates and sodium phosphates) is used as feed.

Experiment start time is at addition of feed solution with stirring of draw on. Observables are the position of the water column in the measuring tubes. Through the measuring tubes a probe is inserted at points-in-time to measure conductivity of either draw or feed solution (Microelectrodes Inc. MI-900 Conductivity electrode, Thermo Scientific Orion 3-Star Conductivity meter.). The rise of the draw column over time is measured and from that flow rates and flux may be calculated appropriately, as mass per area and mass per area per unit of time. Conductivity is measured at both feed and draw in parallel to water column rise.

Supported liquid membranes according to the invention may also take the form of an open or closed sandwich construction, wherein a substantially flat porous filter material provides support on one or both sides of a layer of proteoliposomes, thus immobilising said layer. Examples of filter material are listed in Table 5 below.

**Table 5. List of filters with indication of type, producer and pore selectivities**

| List of abbreviations: MF: microfiltration, NF: nanofiltration, UF: ultrafiltration, RO: Reverse Osmosis, RC: regenerated cellulose, -as: asymmetric, -s: symmetric | | | |
|---|---|---|---|
| **membrane type** | **producer** | **brand name** | **pore specs. size/cut-off Da** |
| RO-as | AlfaLaval | RO98pHt | $\geq$97% NaCl rejection at 15.5 bar. 0.1-2 nm |
| NF-as | AlfaLaval | NF-99 | min. 0.3 kDa $\geq$96% MgSO4 rejection at 9 bar appr.1-10 nm |
| (MF)-s, unlaminated | Sterlitech | PTFE | 0.200 $\mu$m |
| (MF)-as | Sterlitech | PTFE | 0.200 $\mu$m |
| NF-s | Sterlitech | PTFE | 0.450$\mu$m |
| NF-as | Dow Chemical | FilmTec NF270 | appr. 1-10 nm |
| UF-as | AlfaLaval | ETNA 1 kDa | 1 kDa / 2nm |
| UF-as | AlfaLaval | ETNA 10 kDa | 10 kDa/ 6nm |
| MF-s | Millipore | Durapor | 0.450 $\mu$m |
| RC-as | AlfaLaval RC | RC70PP | 10 kDa/ 6nm |

In addition to the specific embodiments of the present invention as illustrated by the figures herein the liquid membrane system of the invention is useful in a biosensor application for detection of compounds having a biochannel modulating effect, such as inhibition and activation, and for drug screening. An example would be incorporation of a functional potassium channel in an immobilised proteoliposome preparation, and screening compound libraries for inhibition or blocking of the channel. Judge SI and Bever CT have shown this principle for the identification of drugs that are useful in the treatment of multiple sclerosis, cf. references section.

**Experimental section**

**Example 1. Preparation of Aquaporin-BLM/ELM: Proteoliposomes**

**Proteoliposome preparation**

Purified SoPIP2;1 was obtained according to the methods described by Maria Karlsson et al. (FEBS Letters 537 (2003) 68-72) and reconstituted into vesicles by mixing with DOPC (1,2-dioleoylphosphatidylcholine) lipid vesicles (10 mg/ml) solubilized in 1% OG (detergent, octylglucoside) at a lipid-to-protein molar ratio (LPR) of 200 in Phosphate buffer (PBS) 10 mM, NaCl 150 mM, pH 7.5. The mixture was dialyzed against phosphate buffered saline buffer in a Float-A-LyzerO G2 Dialysis Cassettes (Spectrum Laboratories Inc, CA, USA) with a molecular cut-off of 8-10.000 Da at room temperature for 2 days with two buffer changes per day (minimum 1:1000 volume sample: volume dialysis buffer). Control vesicles were made in the same manner without protein.

**Bulk liquid membrane preparation**

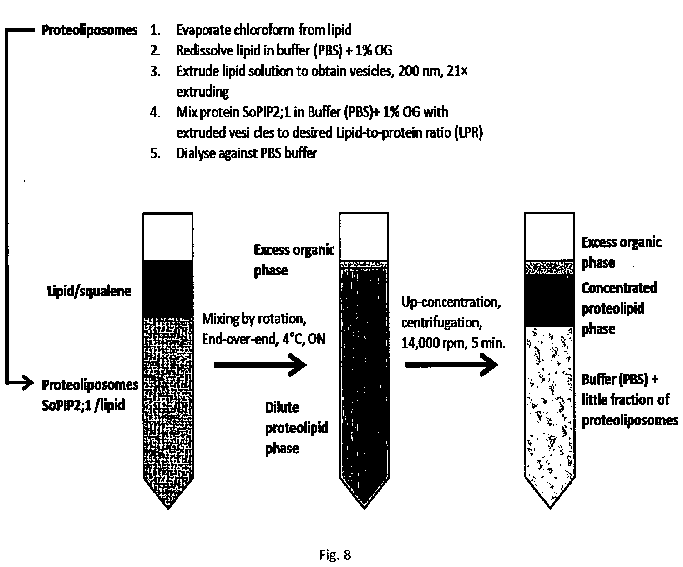

To SoPIP2;1 proteoliposomes prepared as described above was gently added a lipid suspension consisting of DOPC dissolved in squalene in a ratio of 1:5 proteoliposomes: lipid suspension without mixing. This was placed on end-over-end over night at 4 degree Celsius. The resulting bulk liquid membrane emulsion may be used directly or may be up-concentrated by centrifugation at 14.000 rpm for 10 min and subsequently using the middle phase of the resultant three phases solution (top phase: lipid/squalene, middle phase: protein/lipid/squalene, bottom phase: phosphate buffered saline solution). Store at 4 °C until use. The principle of this example is illustrated in Fig. 8.

**Example 2. Preparation of lipid mixture for solvent less aquaporin BLM**

**Materials and Chemicals**

Phospholipids (DOPC), glycerides (mono-oleoyl-glyceride), squalene, linoleic acid (both stored at +5 °C), pentane, labelled phospholipid (e.g. Texas Red® DHPE, Sigma Aldrich).

**Equipment**

Vacuum dessicator, standard lab equipment, water suction flow.

**Required laboratory working time**

1 hour + overnight for storage

**Preparation steps for lipid mixture/solution where lipid / fatty acid / squalene ratio is 1 / 6 / 35**

1) dry down 10 mg lipid from chloroform stock under $N_2$, put under vacuum 30 min
2) add 200 $\mu$L of squalene
3) add 20 $\mu$L of linoleic acid (use Hamilton and pipette through septum)
4) whirlimix gently, preferable under flow of $N_2$
5) add 300 $\mu$L pentane, whirlimix, or alternative 5). If lipid-label is used, then use the chloroform-phase of the labelled lipid (normally around 50 $\mu$L) to mix the ternary component phase. Continue as below, removing the chloroform.
6a) evaporate pentane under flow of $N_2$, then under vaccum until pentane is gone
6b) freeze-thaw samples 5 times in ethanol and dry ice
7) degas emulsion under water suction pump
8) $N_2$-gas on top, put cap and parafilm on and label
9) store at -80 °C overnight.

The solvent free and solvent less lipid mixtures prepared herein are especially suited for incorporation of amphiphilic, transmembrane proteins, such as aquaporins. The proteoliposome preparation of Example 1 can be added to the solvent less lipid mixture according to the procedure mentioned in Example 1.
The BLM preparations of Examples 1 and 2 may be used in the following applications.

After separating the BLM emulsion phase as shown in Fig. 8 this may be deposited on a filter unit, such as a Centriprep® filter device taking due care to completely cover the filter disk optionally using an excess volume. The filter device is now ready for use in extraction of pure water from an aqueous medium providing that an osmotic pressure difference or gradient is established across the filter disk with the deposited aquaporin BLM. Fig. 8 shows the relation between liquid membrane formulation and concentration of the proteoliposome microemulsion (liquid membrane) prepared as described in the experimental section, e.g. Example 1. Basically the liquid membrane is formed through the successive stages: Chloroform is evaporated from the lipid, the lipid is redissolved in buffer (PBS) + 1% OG (detergent), and the lipid solution extruded up to about 20 times using, e.g. a LIPEX barrel extruder or the like through a 200 nm polycarbonate filter to obtain uniform vesicles of about 200 nm diameter. The protein SoPIP2;1 is mixed in buffer (PBS)+ 1% OG with the extruded vesicles to the desired lipid-to-protein molar ratio (LPR), Dialyse $\geq$ 48h against PBS buffer.

**Example 3. Preparations of an aquaporin proteoliposome and control oil/water emulsion using squalene as stabiliser or oil phase and DOPC as amphiphilic lipid.**

**Materials and Chemicals**

Proteoliposome sample and large unilamellar lipid (LUV) sample (use same lipid as in proteoliposome formulation).

Squalene (min. 98% purity as Sigma-Aldrich S3626).

Phospholipid, such as DOPC in chloroform or as a dry powder (Avanti Polar Lipids Inc.); use same lipid species for control emulsion as in proteoliposome formulation. Flourescent lipid probe: 2-dioleoyl-sn-glycero-3-phosphoethanolamine-N-(7-nitro-2-1,3-benzoxadiazol-4-yl (NBD-PE), (Avanti Polar Lipids Inc.)

**Special equipment**

Mini LabRoller (Labnet). 1.5 mL microtubes, sterile.

**Required laboratory working time**

20 min workload and an additional incubation time of approximately 60 min + overnight; making it a total sample preparation time of app. 18 hours.

**Preparation of liposomes (LUV)**

1. Mix 20 ml of PBS with 0.26 g OG to a concentration of 1.3%.
2. In 10 ml PBS/OG add 100 mg asolectin to a concentration of 10 mg/ml.
3. Stir well.
4. Extrude 11 times using a lipid extruder.

**Preparation of proteoliposomes**

1. From the amount of extruded liposomes and the concentration of protein in the stock received, add protein to achieve the desired concentration of protein in the proteoliposomes
2. Dialyze for at least 20 hrs.

Preparation of a squalene oil phase batch

Preparing a 2 mL squalene with 10 mg/mL phospholipid and flourescently labelled lipid.

1. In an 8 mL glass vial weigh off 20mg of asolectin.
2. Add 50$\mu$L of DPhPE/TR in chloroform (0.33mg/ml) and 50 $\mu$L of pure chloroform.
3. Leave for 30 min to rehydrate and dissolve.
4. Add 2 mL squalene and vortex vigorously for 10 min.
5. Put sample under flow of $N_2$-gas (30-45 min).

6. Put sample in desiccators under vacuum for 1 hrs.

7. Cap and store the sample at 4°C until use.

**Preparation of oil phase (squalene) batch**

Preparation of 2 mL squalene with 10 mg/mL phospholipid and fluorescently labelled lipid:

1. In a 6 mL glass vial add 2 mL 10 mg/mL phospholipid in chloroform
2. Evaporate solvent under flow of $N_2$-gas (30-45 min) to obtain a lipid film, or alternatively
3. when using phospholipid powder add 50 $\mu$L chloroform to 20 mg phospholipid to obtain 2 mL of liquid phospholipid.
4. Add 100 $\mu$L of a 1 mg/mL NBD-PE (chloroform) to lipid film from 2. (make sure lipid film is dissolved) or to liquid lipid from 3.
5. Add 2 mL squalene (Sigma-Aldrich) - mix gently
6. Put sample under flow of $N_2$-gas (30-45 min) and afterwards in dessicator under vacuum
7. Gently flow $N_2$-gas across sample before putting cap on, parafilm, and store at -80 °C.

**Preparation of an oil/water SoPIP2;1 or FomA proteoliposome emulsion**

- Volumetric ratio: 3:1 proteoliposome : squalene (= 25 % vol/vol o/w.)
- Allow batches to thermally equilibrate at ambient temperature before use.

1. Add in a 1.5 mL microtube:

- 200 $\mu$L squalene-lipid batch (oil phase batch)
- 600 $\mu$L proteoliposome batch prepared as described in Ex. 1.

2. Put sample at Mini LabRoller and roll sample overnight in refrigerator (top shelf, at app.T=8-10°C)

**Preparation of an oil/water control proteoliposome emulsion**

- same as above using a LUV batch instead of proteoliposome batch

3. After use aerate the liposome and squalene-lipid batches with a flow of $N_2$-gas. Liposome batches must be stored in refrigerator at 5-10°C and the squalene-lipid batch in the -80 °C freezer.

- The squalene-lipid oil phase batch will last for 10 emulsion preparations
- Materials use for a set of emulsion: 1.2 mg aquaporin (SoPIP2;1) or FomA, 40 mg phospholipid, 400 $\mu$L squalene.

**Example 4. Solventless preparation of an AQP oil/water proteoliposome emulsion using squalene as a stabilizer or oil phase and asolectin as amphiphilic lipid. This example provides an alternative method of preparing proteoliposomes (or GPV) emulsions.**

**Materials and Chemicals**

Squalene (Pan Asian Marketing Co. Ltd)
Phospholipid: Asolectin from soybean from Fluka (#11145)
Fluorescent lipid probe: DPhPE/TR (Avanti Polar Lipids Inc.)

**Equipment** (other than standard laboratory equipment)

Mini LabRoller™ (Labnet)
Round bottom test tubes
LIPEX™ Extruder (Northern Lipids Inc.)

**Required laboratory working time**

Day 1: 2.5 hrs. preparation time + lab rolling O.N., in total app. 19.5 hrs.

**Preparation of liposomes**

1. Mix 20 ml of PBS with 0.26 g OG to a concentration of 1.3%.
2. In 10 ml PBS/OG add 100 mg asolectin to a concentration of 10 mg/ml.
3. Stir well to produce liposomes.
4. Extrude liposomes 11 times to create a mono-disperse liposome dispersion.
5. Alternative to step 4.: Exclude step 4 and use liposomes directly without extruding.

**Preparation of proteoliposomes**

1. From the amount of extruded liposomes and the concentration of protein in the stock received , add protein to achieve the desired concentration of protein in the proteoliposomes
2. Dialyze for at least 20 hrs.

Preparation of a squalene oil phase batch

Preparing a 2 mL squalene with 10 mg/mL phospholipid and flourescently labelled lipid.

1. In a 8 mL glass vial weigh off 20mg of asolectin.
2. Add 50 $\mu$l of DPhPE/TR in chloroform (0,33mg/ml) and 50$\mu$l of pure chloroform.
3. Leave for 30 min to rehydrate and dissolve.
4. Add 2 mL squalene and vortex vigorously for 10 min.
5. Put sample under flow of $N_2$-gas (30-45 min).
6. Put sample in desiccators under vacuum for 1 hrs.
7. Cap and store the sample at 4°C until use.

**Preparation of an oil/water AQP proteoliposome emulsion**

-   Volumetric ratio: 3:1 proteoliposome : squalene
-   Allow batches to thermally equilibrate at ambient temperature before use.

    1. Add in a 8 mL glass flask (round bottom):

-   600 $\mu$L squalene-lipid batch (oil phase batch)
-   1800 $\mu$L proteoliposome batch

    2. Put sample at Mini LabRoller and roll sample overnight in darkness
    3. Next day visualize under microscope for quality control

**Example 5. Application of BLMs as prepared above**

The BLM preparations of the invention can suitably be incorporated in a hollow fiber module designed for concentration driven liquid-liquid mass transfer, e. g. a Liquid-Cel extra-flow 10x28 contactor as described in section 4.21 and shown in Figure 4.1(b) in Manuel Aguilar & Jose Luis Cortina "Solvent Extraction and Liquid Membranes", CRC Press, 2008, the contents of which is incorporated herein. The liquid membrane emulsion of the invention can be incorporated in the microporous hollow fibre membranes, and using salt water as the feed fluid and a suitably concentrated draw fluid pure or desalinated water can be extracted from the salt water feed.

**Example 6. Preparation of unilamellar liposomes and SoPIP2;1 proteoliposomes**

Liposomes for protein reconstitution were prepared by evaporation of the chloroform from 20 mg lipid by nitrogen gas, followed by drying in under vacuum in a glass desiccator for 2 h followed by rehydration in 2 mL PBS containing 1% OG, pH 7.4 to a fluid having a lipid concentration of 10 mg/ml. The fluid was extruded 21 times through a 200 nm polycarbonate filter using a LIPEX barrel extruder (Northern Lipids Inc., Burnaby, BC, Canada) to produce liposomes.

The spinach aquaporin SoPIP2;1 protein was obtained from Professor Per Kjellbom and Urban Johansson at The Department of Biochemistry at Lund University in Sweden, and was expressed and purified according to Törnroth-Horsefield *et al.* 2006 (Susanna Törnroth-Horsefield et al. 2006. Structural mechanism of plant aquaporin gating, vol 439, Nature, pp.688-694).

The bacterial aquaporin-Z from *E. Coli* was obtained for Associate professor Jaume Torres, Division of Structural & Computational Biology, School of biological Sciences, Nanyang Technical University, Singapore. Functional aquaporin-Z was overproduced in *E.Coli* strain BL21(DE3) bacterial cultures as His-tagged protein with a tobacco etch virus cleavage site. The fusion protein has 264 amino acid and a Mw of 27234 Da. Genomic DNA from *E. coli* DH5$\alpha$ was used as a source for amplifying the AqpZ gene. The AqpZ gene was amplified using gene specific primers with the addition of a tobacco etch virus cleavage site (TEV); ENLYFQSN at the N-terminus of AqpZ. The amplified AqpZ was digested with the enzyme *Nde*I and *BamH*I and then ligated to the similarly digested 6-His tagged expression pET28b vector DNA. The positive clones were verified by PCR-screening. The authenticity of the constructs was then confirmed by DNA sequencing.

The *E. coli* strain BL21(DE3) was used for expression of the protein. Luria Broth cultures containing 50 $\mu$g/ml kanamycin were incubated for 13-16 hours at 37°C, diluted 100-fold into fresh LB broth and propagated to a density of about 1.2-1.5 (OD at 600 nm). Expression of recombinant protein was induced by addition of 1 mM IPTG for 3 hour at 35°C before centrifugation.

Harvested cells were resuspended in ice-cold binding buffer (20 mM Tris pH 8.0, 50 mM NaCl, 2 mM $\beta$-mercaptoethanol, 10% glycerol) in the presence of 0.4 mg/ml lysozyme, 50 units Bensonase and 3% n-octyl $\beta$-D-Glucopyranoside. The sample was subjected to five times lysis cycles in a microfluidizer at 12,000 Pa. Insoluble material was pelleted by 30 minutes centrifugation at 40,000 x g. The supernatant was passed through a Q-sepharose fast flow column (Amersham Pharmacia), and the flow through was collected. The flow though fraction was topped up with NaCl to 300 mM before loaded onto a pre-equilibrated Ni-NTA column. The column was washed with 100 column volumes of a wash buffer (20 mM Tris pH 8.0, 300 mM NaCl, 25 mM imidazole, 2 mM $\beta$-mercaptoethanol, 10% glycerol) to remove non-specifically bound material. Ni-NTA agarose bound material was eluted with five bed volumes of elution buffer (20 mM Tris pH 8.0, 300 mM NaCl, 300 mM imidazole, 2 mM $\beta$-mercaptoethanol, 10% glycerol, containing 30 mM n-octyl $\beta$-D-Glucopyranoside). AqpZ was further purified with anion exchange chromatography; monoQ column (GE healthcare). The mixture sample was diluted and concentrated to bring the salt and imidazole concentration to approximately 10 mM with Amicon concentrator, membrane cut off 10,000 Da before loading to MonoQ column. The buffer used during anion exchange chromatography were (A) 20 mM Tris pH 8.0, 30 mM OG, 10% glycerol and (B) 20 mM Tris pH 8.0, 1 M NaCl, 30 mM OG, 10% glycerol. The eluted peak fractions containing AqpZ from the ion exchange column was pooled. The purified AqpZ was kept frozen at -80°C.

**Fluorescent labeling of spinach SoPIP2;1 and E.Coli Aqp-Z aquaporins**

Aquaporin transmembrane proteins, spinach aquaporin SoPIP2;1 or *E. Coli* AqpZ, were labeled with badan™. Synthesis and handling of badan™-derivatized proteins was carried out under dim light. To carry out the reaction, 10-fold molar excess of badan™ to SoPIP2;1 from a 20 mM stock solution of badan™ (dissolved in dimethylformamide) to a 10 mg/ml protein solution. The reaction was allowed to take place for 20 h at 4°C with end-over-end rotation. The reaction mixture was desalted for SoPIP2;1 into PBS, 1% OG, 1% glycerol, pH 7.4 and for Aqp-Z into 20 mM Tris, 30 mM OG, pH 8 on a polyacrylamide gel Econo-Pac 10DG desalting column (Bio-Rad). The resulting fluorescently-labeled aquaporins were stored at 4°C until use.

The badan™ labeled SoPIP2;1 or AqpZ were reconstituted by mixing of protein mixture with the liposomes at a lipid-to-protein molar ratio (LPR) of 200. Protein concentrations were determined by UV/Vis absorbance spectroscopy using the extinction coefficient at 280 nm of 46660 M$^{-1}$ cm$^{-1}$ for SoPIP2;1 and 36370 M$^{-1}$ cm$^{-1}$ for AqpZ. The mixed protein and liposome solution was dialyzed for 24 h in a dynamic microdialyzer dialysis device (Spectrum Laboratories Europe, Breda, The Netherlands) using a molecular weight cut-off of 10,000 Daltons and a dialysate flow of 3 ml/min. Control vesicles were made in the same manner without protein. The resulting proteoliposomes or liposomes were stored at 4°C until use.

**Preparation of giant protein vesicles**

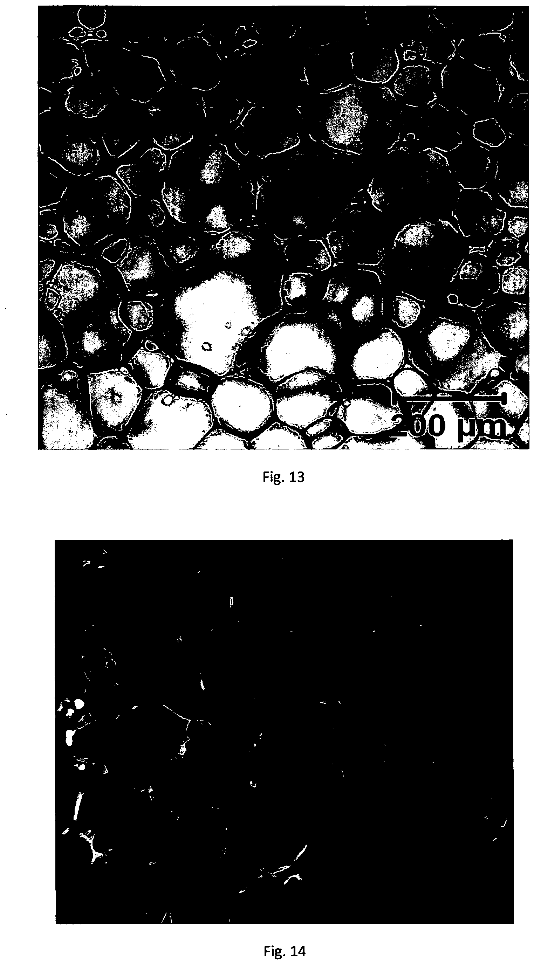

Giant protein vesicles (GPVs) were formed by mixing unilamellar proteoliposomes with a solution of solvent or oil phase and lipid, said oil phase consisting of, e.g., decane or squalene in lipid (10 mg/ml) containing solutions matching that of the proteoliposomes used, e.g. when DOPC is the lipid used for the proteoliposomes, then DOPC is used in the solution. To form GPVs, the solvent or oil phase and lipid solution was gently added to proteoliposomes to a ratio of 1:3 v/v, and the solvent or oil phase and lipid or proteoliposome solutions were mixed with end-over-end rotation over night at 4°C, resulting in the formation of GPVs from the small unilamellar proteoliposomes, cf. Figs. 13 and 14. The GPVs formed are stable compared to lipid vesicles formed in parallel without TM proteins (control vesicles), and have an approximate diameter in the range of 25 $\mu$m to about about 400 $\mu$m. It was observed that the control vesicles were extremely difficult to prepare and were characterised in being without structure and unstable.
The same protocol may be used in the preparation of AqpZ proteoliposomes and giant protein vesicles where a lipid, such as DPhPC is preferably used instead of DOPC.

**Fluorescence spectroscopy and microscopy of badan™-aquaporin**

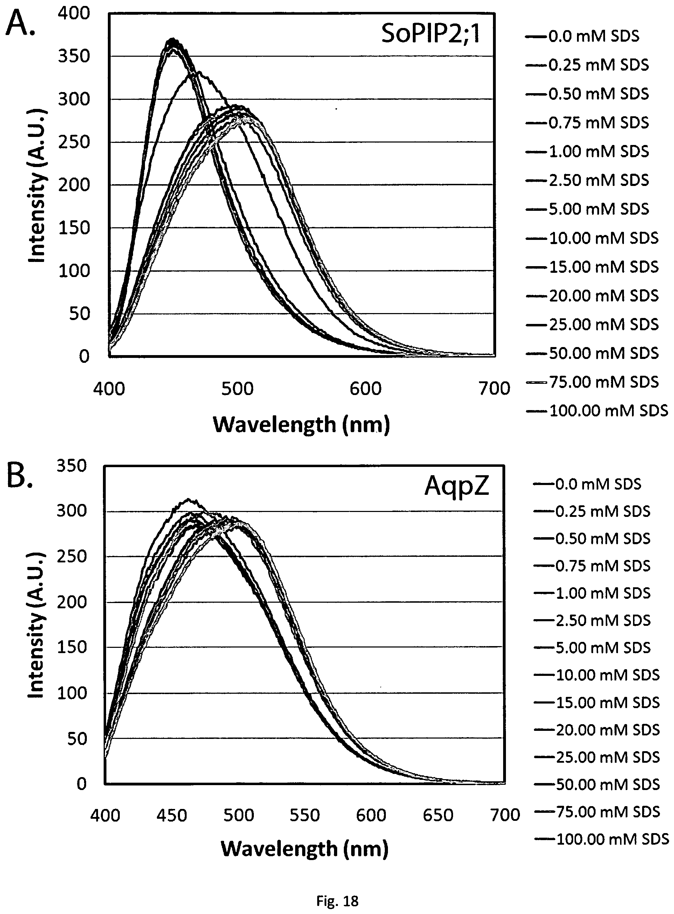

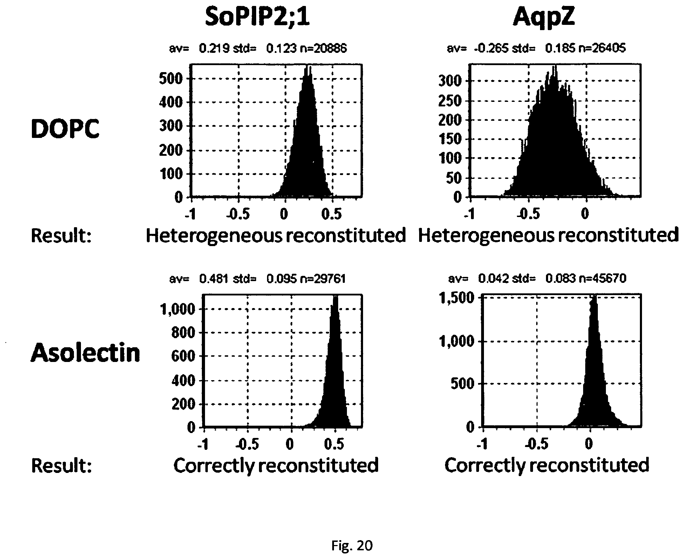

Fluorescence spectroscopy was performed using a Varian Cary Eclipse fluorescence spectrometer (Varian Inc., Palo Alto, CA, USA) with a $\lambda_{ex}$ (excitation wavelength) of 380 nm and emission recorded at 400 to 700 nm.
The fluorescence emission properties of badan™ labeled aquaporin SoPIP2;1 and AqpZ are sensitive to the polarity of the local environment of the fluorescent probe badan. The fluorescence maximum emission yield of badan™ is blue shifted or red shifted if the local environment around the probe becomes more hydrophobic or hydrophilic, respectively. Saturating amounts of SDS causes a red shift in the maximum emission yield. Emission spectral changes can be quantified comparing the generalized polarization (GP) values for shifted and unshifted fluorescence intensity peaks of badan™-labeled aquaporins. GP values were calculated by: GP=$I_b$-$I_g$/$I_b$+$I_g$, where $I_b$ and $I_g$ correspond to the intensities at the blue and green edges of the emission spectrum respectively.
Fluorescence spectroscopy was performed using a Varian Cary Eclipse fluorescence spectrometer (Varian Inc., Palo Alto, CA, USA) with a $\lambda_{ex}$ (excitation wavelength) of 400 nm and emission recorded at 425 to 700 nm. $I_b$ and $I_g$ were calculated from the emission spectra corresponding to the band pass filter range applied for fluorescence confocal microscopy imaging.
GP images covering 420-480 and 505-550 nm fluorescence emission range, respectively, were obtained simultaneously in dual-channel setup on a confocal microscope (model LSM 510 META; Carl Zeiss MicroImaging).
The fluorescence data were analyzed using the Globals software package developed at the Laboratory for Fluorescence Dynamics at the University of Illinois at Urbana-Champaign to obtain the GP image and the associated GP histogram (distribution of the GP values per pixel) (Beechem, J. M.; Gratton, E. In Time-Resolved Laser Spectroscopy in Biochemistry, Proc. of SPIE; Lakowicz, J., Ed. 1988; Vol. 909, p 70-81)
The GP ratio for aquaporin SoPIP2;1 was determined to be +0.4 in PBS, while -0.4 when exposed to 100 mM SDS.
The GP ratio for aquaporin AqpZ, on the other hand, was determined to be +0.0 in PBS, while -0.3 when exposed to 100 mM SDS, cf. Fig. 20.
The SDS makes the fluorescent probe to become more hydrophilic exposed (from PBS to 100 mM SDS), and this is the reason to the shift observed in fluorescence emission wavelengths and thus also the change in GP ratio. We interpret this as GP ratios of around +0.4 or +0.0 for SoPIP2;1 and AqpZ, respectively, correlates to correctly folded protein upon reconstitution. In contrast, GP ratios of around -0.4 or -0.3 for SoPIP2;1 and AqpZ, respectively, correlates to unfolded/incorrectly protein upon reconstitution. GP ratios in between or with broad histograms indicate heterogeneously reconstituted protein (mixture of correctly folded and in-correctly folded protein), cf. Figs. 18, 19 and 20.

**Example 7. Preparation of a stabilized GPV sample**

In the preparation of a 2 mL oil phase with 10 mg/mL phospholipid (same component(s) as in proteoliposome preparation, cf. Example 1, and a fluorescently labelled lipid the following steps are employed:

   1. In a 4 mL glass vial add 2 mL of the phospholipid DOPC (1,2-dioleoylphosphatidylcholine) 10 mg/mL phospholipid (chloroform) (Avant Polar Lipids Inc.)
   2. dry down to lipid film under flow of N$_2$-gas (45 min)
   3. Add 100 $\mu$L of chloroform into which a 1 mg/mL 2-dioleoyl-*sn*-glycero-3-phosphoethanolamine-N-(7-nitro-2-1,3-benzoxadiazol-4-yl (NBD-PE), (Avanti Polar Lipids Inc.) is dissolved (optional labelling) - and dissolve lipid film

4. Add 2 mL of squalene (S3626, Sigma-Aldrich) - mix gently

5. Put sample under flow of $N_2$-gas (45 min)afterwards in dessicator under vacuum for 30 min.

6. Freeze-thaw 5 times using ethanol (96% grade) in liquid $N_2$.

7. Bring sample to ambient temperature and gently flow $N_2$-gas across.

8. Use prepared sample directly or store: put a cap on, parafilm and store at -80 deg

In the preparation of an oil-in-water dispersion of oil phase into an aqueuos proteoliposome phase and formation of a stabilized GPV sample using proteoliposomes and a squalene preparation in the volumetric ratio of proteoliposome : squalene = 3:1, the following steps are employed:

1. Allow batches of proteoliposome (prepared according to Ex. 1) and squalene, prepared under a/, to thermally equilibrate at ambient temperature before use.

2. Add in a 4 mL round bottomed microtubes:

- 400 $\mu$L of prepared squalene sample
- 1800 $\mu$L of prepared proteoliposomes

3. Apply a gentle flow of $N_2$-gas

4. Wrap sample in alu-foil and a laboratory film (Parafilm). Put sample vial at a Mini LabRoller™ Rotator (LabNet International) or similar, and roll sample overnight in fume hood or refrigerator at minimum 10°C.

**Harvesting a stabilized GPV sample**

5. Let sample from 4. equilibrate 30 minutes before further use.

The sample visually exhibits 3 phases: A top phase being an organic phase of squalene, a bottom aqueous phase and an extended third interphase, up to 1200 $\mu$L in volume, enriched by stabilized GPV particles.

6. Remove the interphase, e.g. using a syringe or a separation funnel, and use as a liquid membrane in a relevant water extraction or dialysis unit.

Fig. 14 shows a fluorescence image of aquaporin SoPIP2;1 labeled with the fluorophore 6-bromoacetyl-2-dimethylaminonaphthalene according to the manufacturer's protocol (badan™ manufactured by Molecular Probes, Inc., 29851 Willow Creek Road, Eugene, OR 97402-9132, USA) and reconstituted into giant protein vesicles. The image was acquired using a Zeiss Axioplan2 upright fluorescence microscope (Carl Zeiss, Jena, Germany) equipped with a Roper Cascade cooled frame-transfer CCD monochrome camera. The filter settings used for image acquisition was 390 nm excitation and 435-465 nm emission filters (blue channel). This monochromatic image clearly shows the presence of labeled SoPIP2;1 protein in the vesicle shells (lipid membranes). In addition, both Fig. 13 and 14 show that protein vesicles (GPVs) prepared from unilamellar proteoliposomes are primarily unilamellar. Moreover, the images clearly show the close packing of the GPVs due to the oil phase stabilisation. This is a unique characteristic of the liquid membrane emulsion or vesicle preparation of the invention making it distinctly different from a comparable liposome preparation, cf. microscope images in Figs. 6, 7 and 8 of Norbert Maurer et al. (Biophysical Journal, Volume 80, May 2001,pp 2310-2326) which shows liposomes dispersed without mutual contact in a liquid water phase.

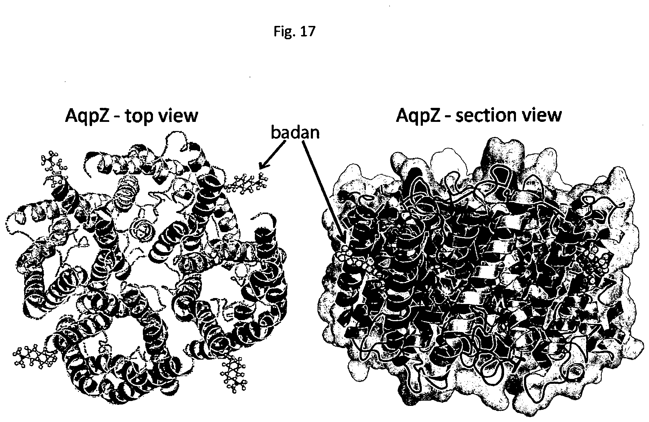

Fig. 17 shows the secondary structure of AqpZ tetramer with four badan™ labels attached. Here the attachment position is on Cys 9 which is located at a protein surface which typically occupies the hydrophobic region of a lipid bilayer membrane.

Fig. 18 shows fluorescence spectroscopy of badan-SoPIP2;1 and badan-AqpZ. Spectra show how the proteins respond to increasing amounts of SDS when the aquaporins are reconstituted into E.Coli total lipid extract lipids. Increased hydrophilicity causes a red shift and a drop in fluorescence emission yield, whereas an increased hydrophobicity leads to a spectra blue shift and a concomitant increase in fluorescence emission yield.

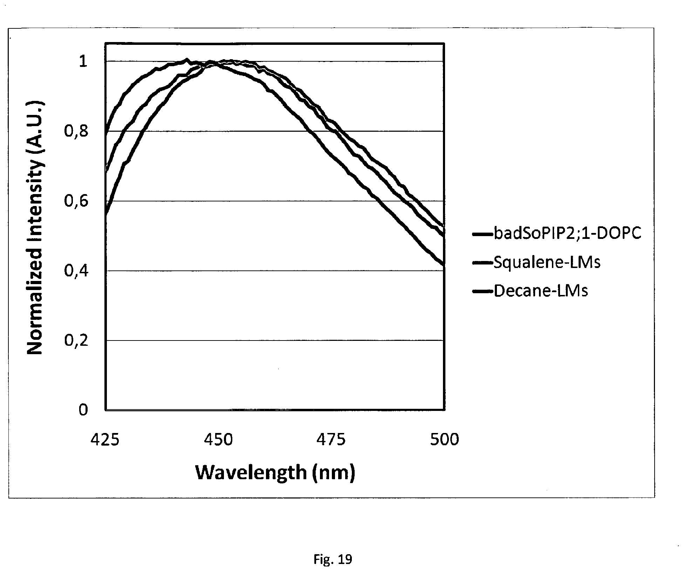

Fig. 19. Sensing the hydrophobicity around badan in SoPIP2;1 liquid membranes (or GPVs) made from oil stabilizers of squalene or decane and compared to proteoliposomes in PBS. The badan-SoPIP2;1 becomes more hydrophobic in decane oil stabilizer since decane has the ability to penetrate into the membrane, and thus increasing the hydrophobicity around the probe of the protein.

**Example 8. Creation of a supported aquaporin liquid membrane prepared by deposition and/or impregnating on a filter substrate.**

The following example method makes use of a centrifugal force spinning down a liquid membrane sample onto a filtering unit which in this case is a microporous PTFE membrane having pore size 0.45 $\mu$m. This example applies to an emulsion of proteoliposome prepared according to Ex. 4.

The following steps apply:

1. Take a Millipore Ultrafree-CL centrifugal filter units, with a PTFE 0.45 micron filter and pipette 400 $\mu$L of the liquid membrane sample into the filter cup and put the filter cup in the filtrate collection tube.
2. Insert the tube into a centrifuge swinging-bucket rotor head.
3. Centrifuge the sample at 2-4000 RPM for 10 min to 30 min allowing the excess water to permeate into the collection tube. A cushioned layer of the liquid membrane oil-water emulsion remains deposited in the PTFE microporous filter.
4. Add a small volume, around 1 mL of PBS buffer.
5. Centrifuge the sample at 400 RPM for 30 min to determine that the added buffer does not permeate into the collecting tube.
6. Remove the added buffer volume

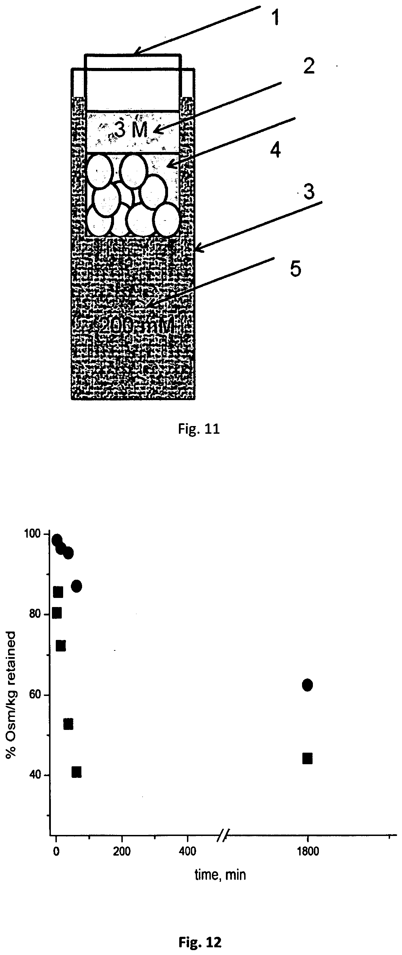

A drawing of a filter unit is shown in Fig. 11, in which (1) Is the filtercup, (2) is the draw solution, (3) is the collection tube, (4) is the liquid membrane (water containing proteoliposomes in an oil phase (squalene), (5) is the feed solution. The concentrations shown, e.g. 3 M for any suitable draw solution and 200 mM for any suitable feed solution are for illustration purposes only.

**Application of the filter unit for forward osmosis**

1. Insert a Millipore microfiltration filter (Millipore Durapore 0.45 micron) on top of the cushioned layer in the filter cup, diameter 10 mm, height 30 mm.
2. Apply an osmotic gradient across the cushioned layer - add a high salt concentrated volume in the filter cup (feed), such as a 3 M potassium chloride solution.
3. Immerse the filter cup in a new collection tube filled with a low osmolarity feed solution content feed solution.
4. Measure osmolarity in the draw, top of filter cup over time.

Fig. 12 is a graph illustrating the degree of measured osmolarity retained in feed in comparison of AQP-containing LM with reference (no AQP present). The graph shows a difference in osmolarity over time between draw and feed solutions. Circular points: AQP liquid membrane, Square points: no AQP in liquid membrane. The graph clearly shows that the AQP LM deposit has a higher ion rejection rate than a non-AQP LM deposit. Osmolarity is obtained by diluting a sample of 25 $\mu$L with 25 $\mu$L of lab water (Millipore filter unit delivering water of 18.2 Mohm·cm conductance) in a sample vial and measuring in a Gonotec Osmomat 030 cryoscopic osmometer.

**Application of filter unit for reverse osmosis**

1/ Insert a Millipore microfiltration filter as millipore Durapore 0,45 micron on top of the cushioned layer in the filter cup. Put a volume of feed water, e.g. at high salt or other osmolyte concentration, to be filtered.
2/ Close the filter cup with a cap with top outlet for a tube.
3/ Place in new collection tube.
4/ Apply pressure (0.2 bar to several bars).
5/ collect permeate and measure its conductivity or osmolarity.

**Example 9. Screening of a forward osmosis configuration with regards to the effect of UF/NF encapsulation membranes and a PBS feed/sorbitol draw combination on flux performance.**

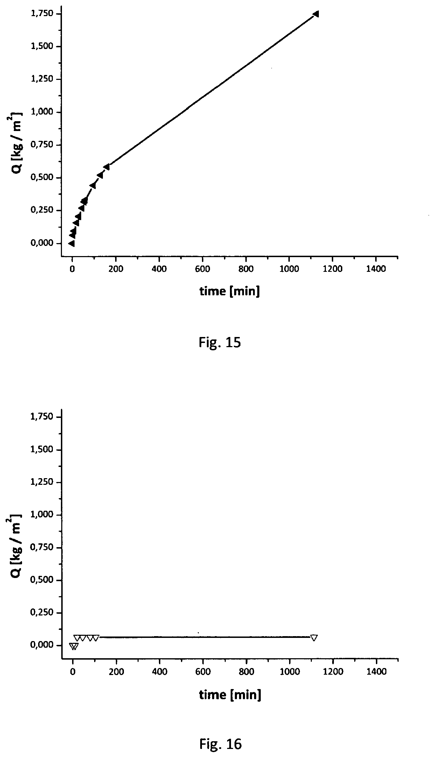

The aim was to test the following encapsulation and feed/draw combination (batch flow cell). We tested the forward osmosis performance of the liquid membrane, LM1, formulation in the batch flow cell in a UF/NF filter sandwiched encapsulations with PBS draw and sorbitol feed solutions. We also used both aquaporin containing samples (SoPIP2;1 PLM), as well as samples without (control-LM.). Results are shown in Figs. 15 and 16.

**Materials**

Encapsulation materials: UF (ETNA 10kDa Alfa Laval)/NF(NF-99 Alfa Laval)
Feed/Draw: PBS/Sorbitol (0.82M)

**List of abbreviations:**

PLM: Protein Liquid Membrane, LLM: Liposome Liquid Membrane, LM: Liquid Membrane,
LM1: Liquid membrane formulation and preparation nr1, SoPIP2: Spinach leaf aquaporin,
UF: ultra filtration membrane, FO: forward osmosis.

**Draw and feed solutions**

The draw solution of D-Sorbitol (Sigma-Aldrich) wsa prepared using pure water with a resistivity of 18.2 MΩ cm (Millipore MilliQ system). A phosphate buffered saline solution (PBS, Sigma-Aldrich) was used as feed.
Final concentration of draw was 0.82 M d-Sorbitol being a solution of 20 bar osmotic pressure (calculated using Morse: $\Pi = iMRT$.) The PBS feed solution ($\sim$ 150 mM NaCl) was at 7 bar osmotic pressure.

**Flow cell and filters**

Batch flow cell according to Fig. 10 was used, and encapsulation filters were prepared and assembled according to standard pre-preparation procedures.
Flat-sheet encapsulation filters, nomenclature and type:

NF: NF-99 (Alfa Laval), UF: ETNA 10kDa (Alfa Laval)

**Liquid membrane samples**

Aqp-SoPIP2 liquid membranes (GPV-LM1), and non-protein LM1 were prepared according to Example 3.

**Flow rates and rejection properties**

Determining permeate transport properties were done according to the equations herein.

**Conclusions**

AQP LM forward osmosis is demonstrated in a batch cell using a D-sorbitol draw solution and PBS feed solution. An initial permeate flux rate of up to 1.3 kg/m2 h was measured after 3 min well above possible flux rates for the UF and NF membranes. A UF/NF (Active layers (skin side) is oriented towards feed and draw, respectively) mode of encapsulation was found to be feasible.
Figure 15 is a graph showing forward osmosis flow QA, area normalized flow, of a SoPIP2;1 GPV formulation. Result obtained in a batch flow cell using a 0.82 M D-sorbitol solution as draw liquid and PBS-buffer (Sigma-Aldrich) as feed. The GPV sample was mounted in a sandwich of UF (towards feed) and NF (towards draw) configuration with the active filter layers towards draw and feed resp.
Figure 16: With same configurations listed for Fig. 15, a non-aquaporin /empty formulation is shown. Some initial flow is observed, and no permeate flow is observed thereafter. In this configuration, the filter membrane facing the feed solution is a UF filter. The LLM-LM1's do not exhibit permeate flow.
This study investigated the performance of PLM1-LM1 forward osmosis using encapsulating membrane filters and draw and feed solutions. One important objective has been to identify a liquid membrane support filter that exerts minimal effect on a forward osmotic draw in our batch cell setup. An osmotically passive encapsulation that keeps the liquid membrane intact allows us to attribute a permeate flow to the activity of aquaporins alone.

**Example 10. Use of the Liquid membrane system of the invention as a biosensor for use in immunological assays and for drug discovery in infectious diseases.**

The major outer-membrane protein of Fusobacterium nucleatum, FomA, is a trimeric protein, which exhibits permeability properties similar to that of other enterobacterial diffusion porins. Each FomA monomer depicts the beta-barrel motif typical of diffusion porins, consisting of 16 antiparallel beta-strands. The FomA porins function as voltage-dependent channel proteins. A liquid membrane emulsion having functional lipid membrane incorporated FomA channels can be prepared according to Example 3 above.

A FomA sensor assay will be constructed as a giant unilammelar vesicle assay and hereafter used as patch clamp device for monitoring sensing. Such a patch-clamp device could for example be an automated patch clamp device developed as a port-a-patch patch clamp device (Nanion Technologies GmbH, Munich, Germany. The FomA porin is a potential drug target which may be useful in drug discovery in Gram-negative bacteria infectious diseases or in Immunological assays. Our preliminary studies have shown that FomA may be blocked by cyclodextrins. This has never previously been described for FomA. The unique feature of cyclodextrin blocking of FomA may be applied to create FomA-based stochastic sensing assays. Certain drugs like anti-depressant drugs may bind to cyclodextrins (Li-Qun Gu et al 2000), which in turn may be registered by the protein, which in this case FomA.

**Example 11. Unilamellar block co-polymer vesicles having incorporated AqpZ (proteopolymersomes)**

The proteopolymersomes were prepared according to Example 2 of WO2009/076174 and as otherwise described therein, and used in the preparation of a bulk liquid membrane instead of proteoliposomes. This bulk liquid membrane can be used in a forward osmosis application according to the present invention, e.g. in applications as described in Fig. 10 and Fig. 11 herein.

The liquid membranes of the invention can be used in various well known contactor modules, such as flat sheet modules and hollow fibre modules, and are not restricted to the uses and applications shown herein. In addition, the liquid membrane emulsions of the invention can be integrated directly in support layers having a suitable pore size distribution. For example, the proteovesicles shown herein having an average diameter of around 200 to 400 or 500 nm are able to be absorbed into the pores of filter membranes having this diameter.

**Example 12. Use of the Liquid membrane system of the invention in a system for haemodialysis.**

The kidneys are organs with important functions in many animals including vertebrates and also some invertebrates. Their function is to remove waste products from the blood, and as such represent a natural filter of the blood. In producing urine, the kidneys excrete wastes such as urea and ammonium; the kidneys also are responsible for the re-absorption of water, glucose, and amino acids.

Each day 180 L of water enters the kidneys, and almost all that water volume is reclaimed (ca. 0.5 L excreted). The salt concentration of urine can be as much as 4 times higher that of blood. The reasons for water reclamation and salt up-concentration in urine are related to the architecture of the kidneys and the function of aquaporins. The kidneys function as a sophisticated forward osmosis system. In the kidney, the thin ascending limb, the thick ascending limb and distal tubule are highly water impermeable, while the other segments are water permeable. This creates a salt gradient across the kidney which is the driving force for the osmosis processes that is necessary for normal renal function.

In this context, aquaporins are abundant in the proximal tubule and the collecting duct. The latter is responsible for water re-absorption and up concentration of the salt in urine compared to that of blood. In renal failure the kidneys fail to function adequately, and may be due to a number of medical problems. Haemodialysis is a medical method for removing waste products such as ions (e.g. $K^+$ and $PO_4^{3-}$) and urea, as well as free water from the blood of a patient with renal failure.

In haemodialysis, a sterilized dialysis solution of mineral ions is used in a forward osmosis process to remove said waste products through a semi-permeable membrane. However, excess water is simultaneously removed from the blood and this must be replenished. Thus, purified water is necessary in haemodialysis. In addition, dialysis patients are exposed to vast quantities of water which is mixed with dialysate concentrate to form the dialysate, where even trace mineral contaminants or bacterial endotoxins can filter into the patient's blood. Even very low concentrations of metal ions, such as aluminium ions stemming from glass ware, as well as low levels of endotoxins, have all caused problems in this regard. For this reason, water used in haemodialysis is carefully purified before use. One purification step involves forcing water through a microporous reverse osmosis membrane. In this way small solutes such as electrolytes are filtered off. Final removal of leftover electrolytes may be done by passing the water through a tank with ion-exchange resins, which remove any leftover anions or cations and replace them with hydroxyl and hydrogen molecules, respectively, leaving ultrapure water.

Even this degree of water purification may be insufficient. The trend lately is to pass this final purified water (after mixing with dialysate concentrate) through a dialyzer membrane. This provides another layer of protection by removing impurities, especially those of bacterial origin that may have accumulated in the water after its passage through the original water purification system.

There are at least two useful applications of the liquid membrane system of the invention in improvement of haemodialysis methods:

1. Production of ultrapure water as described herein can replace the very elaborate systems for water purification that are in use in haemodialysis.

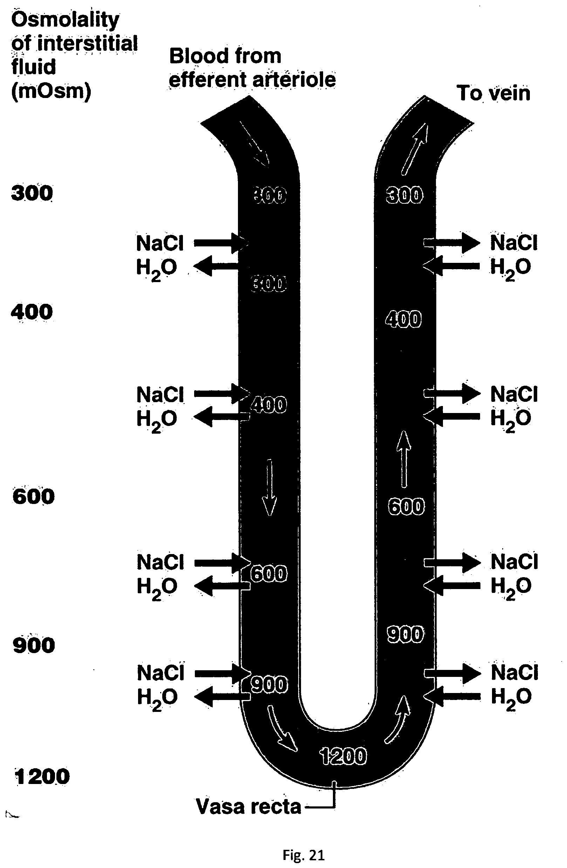

2. Following the forward osmosis process described above where large amounts of water stemming from the patient's blood plasma is simultaneously removed, this may be extracted using an aquaporin liquid membrane in any of the methods described herein. For this purpose, a salt gradient and a counter-current will be created mimicking the normal kidney function across the liquid membrane of the invention, which will then constitute the necessary driving force for the forward osmosis processes, cf. Fig. 21. This will ensure re-use of the patient's own plasma water and eliminate the risks from contaminants present in external water, however purified it may be.

**References:**

US4360448 (A) "Water in oil emulsions useful in liquid membrane", Publication date: 1982-11-23, Inventor(s): Li Norman N; Cahn Robert P; Shrier Adam L, Applicant(s): Exxon Research Engineering Co.

WO/1987/002380 "Production of Low-Ethanol Beverages by Membrane Extraction", Publication Date: 23.04.1987, Inventor: MATSON, Stephen, L, Applicant: SEPRACOR, INC. [US/US]; 33 Locke Drive, Marlborough, MA 01752 (US).

WO/2009/076174 "highly permeable polymeric membranes", publication date: 18.06.2009, inventors: KUMAR, Manish, CLARK, Mark, M., ZILLES, Julie, BRZELAKOWSKI, Mariusz, NEHRING, Rainer, MEIER, Wolfgang.

Tamir Gonen and Thomas Walz, Quarterly Reviews of Biophysics (2006), 39:4:361-396, Cambridge University Press.

Cath et al., Journal of Membrane Science, 281 (2006) 70-87. http://sschi.chtf.stuba.sk/MembraneLab/Equipment.htm .

McGinnis and Elimelech, Desalination, 207 (2007) 370-382.

Quirin Schiermeier, "Purification with a pinch of salt", Nature, 452, 20 March 2008.

Manuel Aguilar & Jose Luis Cortina "Solvent Extraction and Liquid Membranes", CRC Press, 2008.

Judge SI, Bever CT (July 2006). "Potassium channel blockers in multiple sclerosis: neuronal Kv channels and effects of symptomatic treatment". Pharmacol. Ther. 111 (1): 224-59.

Karlsson, M. et al. FEBS Letters 537 (2003) 68-72.

Analytical and bioanalytical chemistry [1618-2642] Hansen yr:2009 vol:395 iss:3 pg:719. Preliminary studies of seawater desalination using forward osmosis. Low, S. C. Desalination and Water Treatment (2009), 7(1-3), 41-46.

Norbert Maurer et al., Biophysical Journal, Volume 80, May 2001, pp 2310-2326

L.D. Mayer et al. "Vesicles of variable sizes produced by a rapid extrusion procedure", Biochimica et Biophysica Acta 858 (1986) 161-168

D.G. Hunter and B.J. Frisken, "Effect of Extrusion Pressure and Lipid Properties on the Size and Polydispersity of Lipid Vesicles", Biophysical Journal 74(6) 1986, 2996-3002

B L Mui et al."Osmotic properties of large unilamellar vesicles prepared by extrusion." Biophysical Journal 64(2) 1993, 443-453.

D. Deamer and A. D. Bangham, Biochimica et Biophysica Acta (BBA) - Biomembranes Volume 443, Issue 3, 7 September 1976, Pages 629-634

Szoka, F. & Papahadjopoulos, D. (1980) Annu. Rev. Biophys. Bioeng. 9, 467-508)

**Claims**

1. A liquid membrane system in the form of a bulk liquid membrane (BLM) containing a biochannel, an emulsion liquid membrane (ELM) containing a biochannel, or a supported (immobilised) liquid membrane (SLM), containing a biochannel,
   wherein the liquid membrane system comprises vesicles, said vesicles being formed from one or more amphiphilic compounds, said amphiphilic compounds forming a bilayer into which the biochannels have been incorporated, wherein said biochannel is an aquaporin water channel, **characterised in that** said liquid membrane system further comprises a stabilising oil phase that comprises squalene, squalane or a mixture thereof.

2. The liquid membrane system according to claim 1, wherein the amphiphilic compounds are lipids.

3. The liquid membrane system according any one of the preceding claims, wherein the biochannels are present in a ratio of 1% to about 70% relative to the vesicle surface area.

4. The liquid membrane system according to any one of the preceding claims which is contained or immobilised in a contactor module.

5. The liquid membrane system of claim 4, wherein the contactor module is selected from the group consisting of a flat sheet module, a hollow fiber separation module, and a spiral wound separation module.

6. The liquid membrane system of claim 4, wherein the contactor module is a two module hollow fiber supported liquid membrane contactor module or a liquid cell extra-flow membrane contactor module.

7. The liquid membrane system according to any one of the preceding claims which is contained or immobilised in a porous support layer.

8. Use of the liquid membrane system according to claim 1 for extracting water from liquid aqueous media by forward osmosis.

9. Use according to claim 8, wherein the liquid medium is salt water and the membrane system is used for desalination of salt water.

10. Use according to claim 8, wherein said forward osmosis process utilizes salt water as the feed solution and a $CO_2/NH_3$ aqueous solution as the draw solution, and where elimination of the dissolved $CO_2$ and $NH_3$ gases is effected through heating to about 58 °C.

11. Use according to claim 8, wherein the water is pure water.

12. Use according to claim 8, wherein the liquid aqueous media is a dialysate resulting from haemodialysis and the liquid membrane system is used in re-extracting water from the dialysate resulting from haemodialysis.

**Patentansprüche**

1. Flüssigkeitsmembransystem in Form einer Flüssigkeitskörpermembran (engl. bulk liquid membrane, BLM), die einen Biokanal enthält, einer Emulsionsflüssigkeitsmembran (engl. emulsion liquid membrane, ELM), die einen Biokanal enthält, oder einer gestützten (immobilisierten) Flüssigkeitsmembran (engl. supported (immobilised) liquid membrane, SLM), die einen Biokanal enthält,
   wobei das Flüssigkeitsmembransystem Vesikel umfasst, wobei die Vesikel aus einer oder mehreren amphiphilen Verbindungen gebildet sind, wobei die amphiphilen Verbindungen eine Doppelschicht bilden, in welche die Biokanäle einbezogen wurden,

wobei der Biokanal ein Aquaporin-Wasserkanal ist, **dadurch gekennzeichnet, dass** das Flüssigkeitsmembransystem ferner eine stabilisierende Ölphase umfasst, die Squalen, Squalan oder ein Gemisch davon umfasst.

**2.** Flüssigkeitsmembransystem nach Anspruch 1, wobei die amphiphilen Verbindungen Lipide sind.

**3.** Flüssigkeitsmembransystem nach einem der vorhergehenden Ansprüche, wobei die Biokanäle, bezogen auf den Vesikeloberflächeninhalt, in einem Verhältnis von 1 % bis etwa 70 % vorhanden sind.

**4.** Flüssigkeitsmembransystem nach einem der vorhergehenden Ansprüche, das in einem Kontaktormodul enthalten oder immobilisiert ist.

**5.** Flüssigkeitsmembransystem von Anspruch 4, wobei das Kontaktormodul aus der Gruppe ausgewählt ist, die aus einem Flachmodul, einem Hohlfaser-Trennmodul und einem spiralförmig gewickelten Trennmodul besteht.

**6.** Flüssigkeitsmembransystem von Anspruch 4, wobei das Kontaktormodul ein zweimodulares hohlfasergestütztes Flüssigkeitsmembran-Kontaktormodul oder ein Flüssigkeitszellen-Extra-Flow-Membran-Kontaktormodul ist.

**7.** Flüssigkeitsmembransystem nach einem der vorhergehenden Ansprüche, das in einer porösen Stützschicht enthalten oder immobilisiert ist.

**8.** Benutzung des Flüssigkeitsmembransystems nach Anspruch 1 zum Extrahieren von Wasser aus flüssigen wässrigen Medien mittels Vorwärtsosmose.

**9.** Benutzung nach Anspruch 8, wobei das flüssige Medium Salzwasser ist und das Membransystem zur Entsalzung von Salzwasser benutzt wird.

**10.** Benutzung nach Anspruch 8, wobei in dem Vorwärtsosmoseverfahren Salzwasser als die Einspeiselösung und eine wässrige $CO_2/NH_3$-Lösung als die Ziehlösung benutzt wird und wobei die Eliminierung der gelösten $CO_2$- und $NH_3$-Gase durch Erhitzen auf etwa 58 °C bewirkt wird.

**11.** Benutzung nach Anspruch 8, wobei das Wasser reines Wasser ist.

**12.** Benutzung nach Anspruch 8, wobei die flüssigen wässrigen Medien ein Dialysat sind, das aus der Hämodialyse resultiert, und das Flüssigkeitsmembransystem beim Rückextrahieren von Wasser aus dem Dialysat, das aus Hämodialyse resultiert, benutzt wird.

## Revendications

**1.** Système membranaire liquide sous la forme d'une membrane liquide volumique (BLM) contenant un biocanal, une membrane liquide en émulsion (ELM) parenthèse contenant un biocanal, ou une membrane liquide supportée (immobilisée) (SLM), contenant un biocanal,
dans lequel le système membranaire liquide comprend des vésicules, lesdites vésicules étant formées à partir d'un de plusieurs composés amphiphiles, lesdits composés amphiphiles formant une bicouche à l'intérieur de laquelle les biocanaux ont été incorporés,
dans lequel ledit biocanal est un canal d'eau d'aquaporine, **caractérisé en ce que** ledit système membranaire liquide comprend en outre une phase huileuse de stabilisation qui contient du squalène, du squalane ou un mélange de ceux-ci.

**2.** Système membranaire liquide selon la revendication 1, dans lequel les composés amphiphiles sont des lipides.

**3.** Système membranaire liquide selon l'une quelconque des revendications précédentes, dans lequel les biocanaux sont présents dans un rapport compris entre 1 % et environ 70 % par rapport à la zone de surface vésiculaire.

**4.** Système membranaire liquide selon l'une quelconque des revendications précédentes qui est confiné ou immobilisé dans un module contacteur.

**5.** Système membranaire liquide selon la revendication 4, dans lequel le module contacteur est choisi parmi le groupe constitué d'un module à feuillet plat, d'un module de séparation à fibres creux, et d'un module de séparation de plaies en spiral.

6. Système membranaire liquide selon la revendication 4, dans lequel le module contacteur consiste en un module contacteur membranaire liquide supporté par des fibres creuses à deux modules ou un module contacteur membranaire par écoulement supplémentaire pour cellule liquide.

7. Système membranaire liquide selon l'une quelconque des revendications précédentes qui est confiné ou immobilisé dans une couche de support poreuse.

8. Utilisation du système membranaire liquide selon la revendication 1 pour l'extraction d'eau à partir de milieux aqueux liquides par osmose directe.

9. Utilisation selon la revendication 8, dans laquelle le milieu liquide est de l'eau salée et le système membranaire est utilisé pour la désalinisation d'eau salée.

10. Utilisation selon la revendication 8, dans laquelle ledit processus d'osmose directe utilise de l'eau salée comme solution d'alimentation et une solution aqueuse de $CO_2/NH_3$ comme solution de puisage, et où l'élimination du $CO_2$ dissout et des gaz $NH_3$ est effectuée par chauffage à environ 58 °C.

11. Utilisation selon la revendication 8, dans laquelle l'eau est de l'eau pure.

12. Utilisation selon la revendication 8, dans laquelle le milieu aqueux liquide est un dialysat résultant de l'hémodialyse et le système membranaire liquide est utilisé dans la réextraction d'eau à partir du dialysat résultant de l'hémodialyse.

Fig. 1

Source phase

Receiving phase

Organic membrane
containing carrier

Stirring bar

Source phase

Organic membrane
containing carrier

Receiving
phase

Source
phase

Receiving
phase

Supported membrane containing
organic liquid and carrier

Fig. 2

Lumen Flow

Header

Fiber wall

Shell flow

Individual Hollow Fibers

Module casing

Shell flow

Header

Lumen Flow

Fiber pores

Fig. 3

Source Phase    Source Phase    Receiving Phase    Receiving Phase

Fiber wall

membrane phase

stirrer

Fiber pores

Each fiber contacts membrane phase

Fig. 4

# Concentration of aqueous solutions

Proteoliposomes with increased volume

Proteoliposomes with aqp's & salt gradient inside vesicle

Solution to be concentrated

Concentrated solution

**(c) phase separator**

Fig. 5

Forward osmosis

Fig. 6

# Forward osmosis – following ultrafiltration of uniform electrolyte.

# Complete application of water extraction from any aqueous solution

Proteoliposomes with aqp's
& salt gradient inside vesicle

Proteoliposomes with increased volume

Proteoliposomes with aqp's
& salt gradient inside vesicle

Concentrated
Solution of
large
Electrolyte/
Dissolved gases

Diluted
Solution of large
electrolyte /dissolved
gases

Waste water

Concentrated
waste water

45 mm

25 mm

(c) phase separator

(c) phase separator

Ultrafiltration
membrane
operating
with low energy
consumption

Clean water

Fig. 7

# ♠ Pressure retarded osmosis for osmotic power production

Each fiber contacts membrane phase

Proteoliposomes 1. Evaporate chloroform from lipid
2. Redissolve lipid in buffer (PBS) + 1% OG
3. Extrude lipid solution to obtain vesicles, 200 nm, 21x extruding
4. Mix protein SoPIP2;1 in Buffer (PBS)+ 1% OG with extruded vesi cles to desired Lipid-to-protein ratio (LPR)
5. Dialyse against PBS buffer

Lipid/squalene

Mixing by rotation,
End-over-end, 4°C, ON

Proteoliposomes
SoPIP2;1 /lipid

Dilute proteolipid phase

Excess organic phase

Up-concentration,
centrifugation,
14,000 rpm, 5 min.

Excess organic phase

Concentrated proteolipid phase

Buffer (PBS) + little fraction of proteoliposomes

Fig. 8

$K_{feed}(t)$

$$\Delta V_Q \ (K_{permeate})$$

$K_{draw}(t)$

Fig. 9

Fig. 10

Fig. 11

Fig. 12

Fig. 13

Fig. 14

Fig. 15

Fig. 16

Fig. 17

AqpZ - top view

AqpZ - section view

badan

Fig. 18

Fig. 19

Fig. 20

Fig. 21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4360448 A
- WO 87002380 A
- US 20090120874 A
- EP 1801572 A
- US 2009308727 A1
- US 20091217 B
- WO 2006122566 A
- WO 2009076174 A
- WO 1987002380 A

### Non-patent literature cited in the description

- **J.O. KESSLER ; C.D. MOODY.** Drinking water from sea water by forward osmosis. *Desalination,* 1976, vol. 18, 297-306
- **J.R. MCCUTCHEON ; R.L. MCGINNIS ; M. ELIMELECH.** Desalination by a novel ammonia-carbon dioxide forward osmosis process: influence of draw and feed solution concentrations on process performance. *J. Membr. Sci.,* 2006, vol. 278 (278), 114-123
- **M. GOULIAN et al.** *Biophysical Journal,* January 1998, vol. 74, 328-337
- **A. VIALLAT et al.** *Biophysical Journal,* April 2004, vol. 86, 2179-2187
- **BOON, M. ; SMITH, BD.** Synthetic membrane transporters. *Current Opinion in Chemical Biology,* 2002, vol. 6, 749-756
- **WHITE S.** *Biophys. J.,* 1978, vol. 23
- **L.D. MAYER ; M.J. HOPE ; P.R. CULLIS.** Vesicles of variable sizes produced by a rapid extrusion procedure. *Biochimica et Biophysica Acta,* 1986, vol. 858, 161-168
- **D.G. HUNTER ; B.J. FRISKEN.** Effect of Extrusion Pressure and Lipid Properties on the Size and Polydispersity of Lipid Vesicles. *Biophysical Journal,* 1986, vol. 74 (6), 2996-3002
- **B L MUI ; P R CULLIS ; E A EVANS ; T D MADDEN.** Osmotic properties of large unilamellar vesicles prepared by extrusion. *Biophysical Journal,* 1993, vol. 64 (2), 443-453
- **D. DEAMER ; A. D. BANGHAM.** *Biochimica et Biophysica Acta (BBA) - Biomembranes,* 07 September 1976, vol. 443 (3), 629-634
- **SZOKA, F. ; PAPAHADJOPOULOS, D.** *Annu. Rev. Biophys.Bioeng.,* 1980, vol. 9, 467-508
- Membrane for filtering of water. **TAMIR GONEN ; THOMAS WALZ.** Quarterly Reviews of Biophysics. Cambridge University Press, 2006, vol. 39, 361-396
- **CATH et al.** *Journal of Membrane Science,* 2006, vol. 281, 70-87
- **MCGINNIS ; ELIMELECH.** *Desalination,* 2007, vol. 207, 370-382
- **QUIRIN SCHIERMEIER.** Purification with a pinch of salt. *Nature,* 20 March 2008, vol. 452
- Quantities, Units and Symbols in Physical Chemistry. INTERNATIONAL UNION OF PURE AND APPLIED CHEMISTRY. 1993
- **STEN-KNUDSEN.** Stoftransport, membranpotentialer og elektriske impulser over biologiske membraner. *Akademisk Forlag,* 1995
- **MARIA KARLSSON et al.** *FEBS Letters,* 2003, vol. 537, 68-72
- **MANUEL AGUILAR ; JOSE LUIS CORTINA.** Solvent Extraction and Liquid Membranes. CRC Press, 2008
- **PROFESSOR PER KJELLBOM ; URBAN JOHANSSON.** Department of Biochemistry. Lund University
- **SUSANNA TÖRNROTH-HORSEFIELD et al.** Structural mechanism of plant aquaporin gating. *Nature,* vol. 439, 688-694
- **ASSOCIATE PROFESSOR JAUME TORRES.** Division of Structural & Computational Biology. Nanyang Technical University
- Time-Resolved Laser Spectroscopy in Biochemistry. **BEECHEM, J. M. ; GRATTON, E.** Proc. of SPIE. 1988, vol. 909, 70-81
- **NORBERT MAURER et al.** *Biophysical Journal,* May 2001, vol. 80, 2310-2326
- **TAMIR GONEN ; THOMAS WALZ.** Quarterly Reviews of Biophysics. Cambridge University Press, 2006, vol. 39, 361-396
- **CATH et al.** *Journal of Membrane Science,* 2006, vol. 281, 70-87, http://sschi.chtf.stuba.sk/MembraneLab/Equipment.htm
- **JUDGE SI ; BEVER CT.** Potassium channel blockers in multiple sclerosis: neuronal Kv channels and effects of symptomatic treatment. *Pharmacol. Ther.,* July 2006, vol. 111 (1), 224-59

- **KARLSSON, M. et al.** *FEBS Letters,* 2003, vol. 537, 68-72
- **HANSEN.** *Analytical and bioanalytical chemistry,* 2009, vol. 395 (3), 719
- **LOW, S. C.** Preliminary studies of seawater desalination using forward osmosis. *Desalination and Water Treatment,* 2009, vol. 7 (1-3), 41-46
- **L.D. MAYER et al.** Vesicles of variable sizes produced by a rapid extrusion procedure. *Biochimica et Biophysica Acta,* 1986, vol. 858, 161-168
- **B L MUI et al.** Osmotic properties of large unilamellar vesicles prepared by extrusion. *Biophysical Journal,* 1993, vol. 64 (2), 443-453
- **SZOKA, F. ; PAPAHADJOPOULOS, D.** *Annu. Rev. Biophys. Bioeng.,* 1980, vol. 9, 467-508